# EUROPEAN PATENT SPECIFICATION

(11) **EP 0 581 501 B1**
(45) Date of publication and mention of the grant of the patent: **09.09.1998**
(21) Application number: 93305608.7
(22) Date of filing: 16.07.1993
(51) Int. Cl.: C07D 477/00, C07F 9/568, C07D 401/12, C07D 401/14, A61K 31/40

(54) **Antibiotic carbapenem compounds**
Antibiotische Carbapenemverbindungen
Dérivés de carbapénème antibiotiques

(30) Priority: 21.07.1992 EP 92402104
(43) Date of publication of application: 02.02.1994
(73) Proprietor: ZENECA LIMITED, London W1Y 6LN (GB); ZENECA Pharma S.A., 95022 Cergy Pontoise Cédex (FR)
(72) Inventor: Jung, Frederic Henri, F-51064 Reims Cedex (FR)
(74) Representative: Denerley, Paul Millington, Dr.

(56) References cited:
- EP-A- 0 126 587
- EP-A- 0 442 497
- EP-A- 0 443 883
- WO-A-92/17481

## Description

The present invention relates to carbapenems and in particular to such compounds containing a carboxy substituted pyridyl group. This invention further relates to processes for their preparation, to intermediates in their preparation, to their use as therapeutic agents and to pharmaceutical compositions containing them. The compounds of this invention are antibiotics and can be used in the treatment of any disease that is conventionally treated with antibiotics for example in the treatment of bacterial infection in mammals including humans.

Carbapenems were first isolated from fermentation media in 1974 and were found to have broad spectrum antibacterial activity. Since this discovery substantial investigations have been made into new carbapenem derivatives and many hundreds of patents and scientific papers have been published.

The first, and so far the only, carbapenem to be commercially marketed is imipenem (N-formididoyl thienamycin). This compound has a broad spectrum of antibacterial activity.

European patent application, publication no. 0216587-A2 discloses thiopyrrolidinyl carbapenem compounds and includes specific compounds in which the pyrrolidine ring is substituted by pyridylcarbamoyl. European patent applications EP 442497-A1 and EP 443883-A1 encompass thiopyrrolidinyl carbapenem compounds in which the pyrrolidine ring is substituted by -CO-Q⁴-R⁴ and -CON(R²)Q respectively, in which Q is a group containing a quaternised nitrogen including, more specifically, N-methylpyridinium.

The present invention provides thiopyrrolidinyl carbapenem compounds wherein the pyrroldine ring is substituted by a carboxypyridylcarbamoyl group. These compounds show a broad spectrum of antibacterial activity including both Gram positive and negative, aerobic and anaerobic bacteria. They exhibit good stability to beta-lactamases. In addition representative compounds of this invention exhibit favourable pharmacokinetics in particular long half life.

The carbapenem derivatives referred to herein are named in accordance with the generally accepted semi-systematic nomenclature.

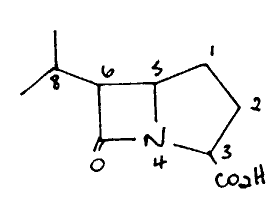

Accordingly the present invention provides a compound of the formula (I) 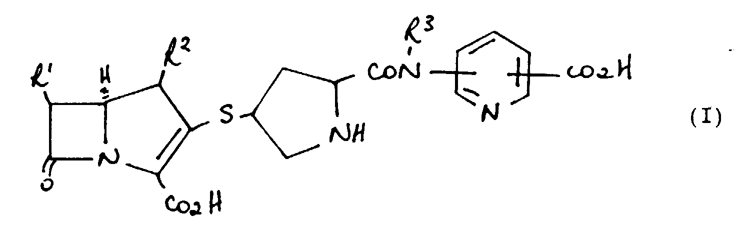 wherein:
R¹ is 1-hydroxyethyl, 1-fluoroethyl or hydroxymethyl;
R² is hydrogen or C₁₋₄alkyl;
R³ is hydrogen or C₁₋₄alkyl;
and the pyridyl group is bonded to the nitrogen of the linking carbamoyl group by a carbon atom, is substituted with the carboxy group on a carbon atom, and is optionally further substituted, on ring carbon atoms, by one or two substituents selected from halo, cyano, C₁₋₄alkyl, nitro, hydroxy, carboxy, C₁₋₄alkoxy, trifluoromethyl, C₁₋₄alkoxycarbonyl, amino, C₁₋₄alkylamino, di-C₁₋₄alkylamino, C₁₋₄alkylS(O)ₙ- (wherein n is 0-2), C₁₋₄alkanoylamino, C₁₋₄alkanoyl(N-C₁₋₄alkyl) amino, carbamoyl, C₁₋₄alkylcarbamoyl and di-C₁₋₄alkylcarbamoyl:
or a pharmaceutically acceptable salt or in vivo hydrolysable ester thereof.

The term alkyl includes all straight and branched chain structures, for example, C₁₋₄alkyl includes n-butyl and 2-methylpropyl.

Preferably R¹ is 1-hydroxyethyl.

R² is hydrogen or C₁₋₄alkyl for example methyl, ethyl, n-propyl, 1-methylethyl and n-butyl.

Preferably R² is hydrogen or methyl and in particular R² is methyl.

R³ is hydrogen or C₁₋₄alkyl for example methyl, ethyl, n-propyl, 1-methylethyl and n-butyl.

Preferably R³ is hydrogen or methyl.

Most preferably R³ is hydrogen.

Suitable substituents for the pyridyl ring include, for example:-

| | |
|---|---|
| for halo | fluoro, chloro and bromo; |
| for C₁₋₄alkyl | methyl, ethyl, propyl, 1-methylethyl,butyl and 2 methylpropyl; |
| for C₁₋₄alkoxy | methoxy, ethoxy, propoxy, 1-methylethoxy, butoxy and 2 methylpropoxy; |
| for C₁₋₄alkylcarbamoyl | methylcarbamoyl, ethylcarbamoyl and propylcarbamoyl; |
| for di-C₁₋₄alkylcarbamoyl | dimethylcarbamoyl and diethylcarbamoyl; |
| for C₁₋₄alkylamino | methylamino, ethylamino and propylamino; |
| for di-C₁₋₄alkylamino | dimethylamino, diethylamino and methylethylamino; |
| for C₁₋₄alkylS(O)ₙ- | methylthio, methylsulfinyl and methylsulfonyl; |
| for C₁₋₄alkanoylamino | acetamido and propionamido; |
| for C₁₋₄alkoxycarbonyl | methoxycarbonyl and ethoxycarbonyl; |
| for C₁₋₄alkanoyl(N-C₁₋₄alkyl)amino | N-methylacetamido and N-ethylacetamido. |

Preferably when the pyridyl ring is optionally substituted, the optional substituents are selected from halo, cyano, C₁₋₄alkyl, nitro, carboxy, hydroxy, C₁₋₄alkoxy, carbamoyl, amino and trifluoromethyl.

The present invention covers all epimeric, diastereoisomeric and tautomeric forms of the compounds of the formula (I) wherein the absolute stereochemistry at the 5-position is as illustrated in formula (I). When a bond is represented as a wedge, this indicates that in three dimensions the bond would be coming forward out of the paper and when a bond is represented as hatched, this indicates that in three dimensions the bond would be going back into the paper. The compounds of the formula (I) have a number of other centres of optical activity, namely: within the group R¹ (when R¹ is 1-hydroxyethyl or 1-fluoroethyl); at the 6-position; at the 1-position (when R² is C₁₋₄alkyl); and at the 2' and 4' positions in the pyrrolidine ring:

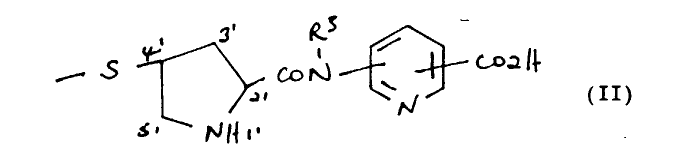

Preferred compounds are those in which the beta-lactam protons are in trans configuration with respect to one another. When R¹ is 1-hydroxyethyl or 1-fluoroethyl it is preferred that the 8-substituent has the R-configuration. Thus a preferred class of compounds is that of the formula (III): 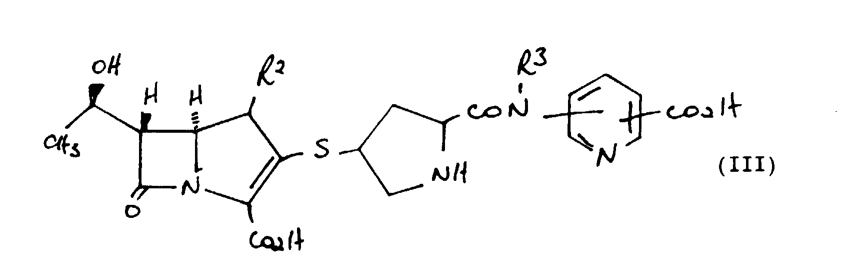 and pharmaceutically acceptable salts and in vivo hydrolysable esters thereof, wherein R², R³ and optional substituents on the pyridyl ring are as hereinbefore defined.

When R² is C₁₋₄alkyl for example methyl it is preferred that the compound is in the form of the 1R configuration.

In one aspect the pyrrolidine ring has the following absolute stereochemistry at the 2'- and 4'- positions:

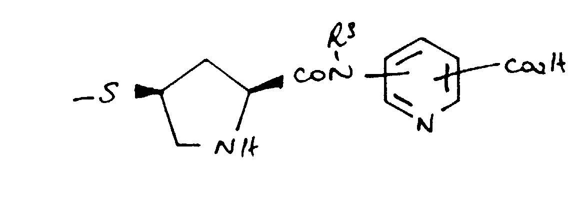

In another aspect the pyrrolidine ring has the following absolute stereochemistry at the 2' and 4' positions:

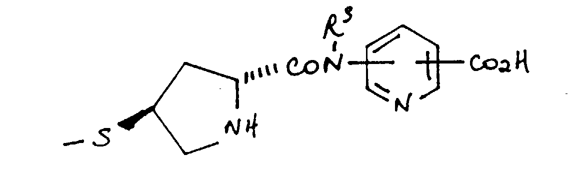

A suitable class of compounds of the present invention is that of the formula (IV): 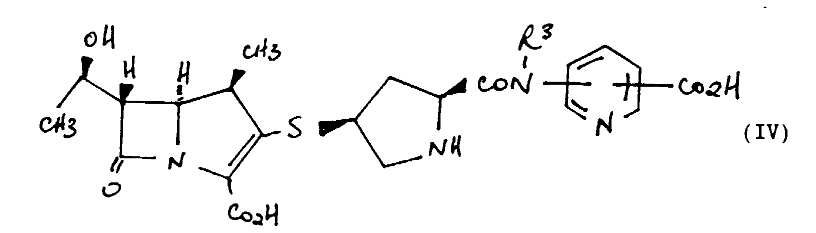 and pharmaceutically acceptable salts and in vivo hydrolysable esters thereof;
wherein R³ and optional substituents on the pyridyl ring are as defined hereinbefore in formula (I).

In another aspect a suitable class of compounds are the compounds of the formula (IVA): 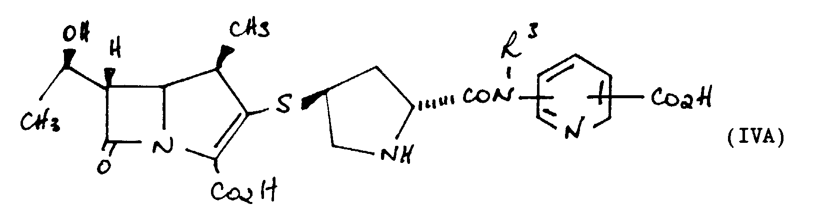 and pharmaceutically acceptable salts and in vivo hydrolysable esters thereof;
wherein R³ and optional substituents on the pyridyl ring are as defined in formula (I).

In another aspect a suitable class of compounds are the compounds of the formula (IV) wherein R³ is hydrogen, methyl or ethyl; and optional substituents on the pyridyl ring are as defined hereinabove in formula (I).

In yet another aspect a suitable class of compounds is that of the compounds of the formula (IV) wherein the pyridyl ring is optionally further substituted by one or two substituents selected from methyl, ethyl, hydroxy, carboxy, cyano, fluoro, chloro, bromo, carbamoyl, nitro, methoxy, ethoxy and propoxy; and R³ is as defined hereinbefore in formula (I).

A particular class of compounds of the present invention is that of the formula (IV) wherein:
R³ is hydrogen or methyl;
and the pyridyl ring is optionally further substituted by one or two substituents selected from methyl, ethyl, hydroxy, carboxy, cyano, chloro, bromo, nitro, methoxy and ethoxy.

A particular class of compounds of the present invention is that of the formula (IVA) wherein:
R³ is hydrogen or methyl;
and the pyridyl ring is optionally further substituted by one or two substituents selected from methyl, ethyl, hydroxy, carboxy, cyano, chloro, bromo, nitro, methoxy and ethoxy.

A preferred class of compounds of the present invention is that of the formula (IV) wherein:
R³ is hydrogen;
and the pyridyl ring is optionally further substituted by one or two substituents selected from methyl, hydroxy, chloro and carboxy.

A preferred class of compounds of the present invention is that of the formula (IVA) wherein:
R³ is hydrogen;
and the pyridyl ring is optionally further substituted by one or two substituents selected from methyl, hydroxy, chloro and carboxy.

A more preferred class of compounds of the present invention is that of the formula (IV) wherein:
R³ is hydrogen;
and the pyridyl ring is not further substituted.

A more preferred class of compounds of the present invention is that of the formula (IVA) wherein:
R³ is hydrogen;
and the pyridyl ring is not further substituted.

Particular compounds of the present invention are, for example, the following compounds of the formula (IV):
(1R,5S,6S,8R,2'S,4'S)-2-(2-(4-carboxy-2-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(3-carboxy-5-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(3-carboxy-2-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(5-carboxy-2-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxy-4-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxy-6-pyridylcarbamoyl)pyrrolidin-4-ylthio)-6-(1-hydroxyethyl)-1-methylcarbapenem-3-carboxylic acid;
(1R,5S,6S,8R,2'R,4'S)-2-(2-(2-carboxy-6-pyridylcarbamoyl)pyrrolidin-4-ylthio)-6-(1-hydroxyethyl)-1-methylcarbapenem-3-carboxylic acid;
and pharmaceutically acceptable salts and in vivo hydrolysable esters thereof.

Suitable pharmaceutically acceptable salts include acid addition salts such as hydrochloride, hydrobromide, citrate, maleate and salts formed with phosphoric and sulfuric acid. In another aspect suitable salts are base salts such as an alkali metal salt for example sodium or potassium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine or aminoacids, for example, lysine.

Preferred pharmaceutically acceptable salts are sodium and potassium. However, to facilitate isolation of the salt during preparation, salts which are less soluble in the chosen solvent may be preferred, whether pharmaceutically acceptable or not.

For the avoidance of doubt there may be one, two, three or four salt-forming cations depending on the number of carboxylic acid functions and valency of said cations.

In vivo hydrolysable esters are those pharmaceutically acceptable esters that hydrolyse in the human body to produce the parent hydroxy or carboxy compound. Such esters can be identified by administering, eg. intravenously to a test animal, the compound under test and subsequently examining the test animal's body fluids. Suitable in vivo hydrolysable esters for hydroxy include acetoxy, propionyloxy, pivaloyloxy, C₁₋₄alkoxycarbonyloxy for example ethoxycarbonyloxy, phenylacetoxy and phthalidyl. Suitable in vivo hydrolysable esters for carboxy include C₁₋₆alkoxymethyl esters for example methoxymethyl; C₁₋₆alkanoyloxymethyl esters for example pivaloyloxymethyl; C₃₋₈ cycloalkoxycarbonyloxyC₁₋₆alkyl, for example 1-cyclohexyloxycarbonyloxyethyl; 1,3-dioxolen-2-onylmethyl esters for example 5-methyl-1,3-dioxolen-2-onylmethyl; phthalidyl esters and C₁₋₆alkoxycarbonyloxyethyl esters for example 1-ethoxycarbonyloxyethyl and may be formed at any carboxy group in the compounds of this invention.

In order to use a compound of the formula (I) or a pharmaceutically acceptable salt or in vivo hydrolysable ester thereof for the therapeutic treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula (I) or a pharmaceutically acceptable salt or in vivo hydrolysable ester thereof and a pharmaceutically acceptable carrier.

The pharmaceutical compositions of this invention may be administered in standard manner for the disease condition that it is desired to treat, for example by oral, rectal or parenteral administration. For these purposes the compounds of this invention may be formulated by means known in the art into the form of, for example, tablets, capsules, aqueous or oily solutions or suspensions, emulsions, dispersible powders, suppositories and sterile injectable aqueous or oily solutions or suspensions.

The compounds of the present invention may be formulated as dry powder filled vials, which may contain the compound of the present invention alone or as a dry blended mixture. For example an acidic compound of the present invention may be dry blended with an alkali metal carbonate or bicarbonate. Freeze dried formulations of compounds of the present invention, alone or as a mixture with standard excipients, are possible. Standard excipients include structure formers, cryoprotectants and pH modifiers, such as, mannitol, sorbitol, lactose, glucose, sodium chloride, dextran, sucrose, maltose, gelatin, bovine serum albumin (BSA), glycine, mannose, ribose, polyvinylpyrrolidine (PVP), cellulose derivatives, glutamine, inositol, potassium glutamate, erythritol, serine and other amino acids and buffer agents e.g. disodium hydrogen phosphate and potassium citrate.

In addition to the compounds of the present invention the pharmaceutical composition of this invention may also contain, or be co-administered with, one or more known drugs selected from other clinically useful antibacterial agents (for example other beta-lactams or aminoglycosides), inhibitors of beta-lactamase (for example clavulanic acid), renal tubular blocking agents (e.g. probenecid) and inhibitors of metabolising enzymes (for example inhibitors of dehydropeptidases, for example Z-2-acylamino-3-substituted propenoates such as cilastatin) and N-acylated amino acids such as betamipron (also see EP-A-178911).

A suitable pharmaceutical composition of this invention is one suitable for oral administration in unit dosage form, for example a tablet or capsule which contains between 100mg and 1g of the compound of this invention.

A preferred pharmaceutical composition of the invention is one suitable for intravenous, subcutaneous or intramuscular injection, for example a sterile injectable composition containing between 1 and 50% w/w of the compound of this invention.

Specific examples of compositions, which are constituted as a 1% solution in water, freeze dried and may be made up by adding 0.9% aqueous sodium chloride solution to give the required concentration, preferably 1mg-10mg/ml, are as follows:

### Composition 1

| | |
|---|---|
| Compound of Example 1 | 50mg |

### Composition 2

| | |
|---|---|
| Compound of Example 1 | 50mg |
| Glycine | 31mg |

Further specific examples of compositions are as above, but where the compound of example 1 is replaced by any one of examples 2 to 7.

The pharmaceutical compositions of the invention will normally be administered to man in order to combat infections caused by bacteria, in the same general manner as that employed for imipenem due allowance being made in terms of dose levels for the pharmacokinetics of the compound of the present invention relative to the clinical use of imipenem. Thus each patient will receive a daily intravenous, subcutaneous or intramuscular dose of 0.05 to 5g, and preferably 0.1 to 2.5g, of the compound of this invention, the composition being administered 1 to 4 times per day, preferably 1 or 2 times a day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose. Thus a suitable daily oral dose is 0.05 to 5g. of the compound of this invention, the composition being administered 1 to 4 times per day.

In a further aspect the present invention provides a process for preparing the compounds of the formula (I) or a pharmaceutically acceptable salt or in vivo hydrolysable ester thereof which process comprises deprotecting a compound of the formula (V) wherein the pyridyl ring is optionally further substituted as in formula (I): 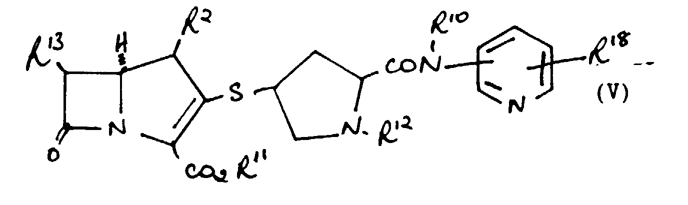 wherein R² is as hereinbefore defined; R¹⁰ is a group R³ or an amino protecting group; R¹³ is a group R¹, protected hydroxymethyl or 1-(protected hydroxy)ethyl; R¹¹ is hydrogen or a carboxy protecting group; R¹² is hydrogen or an amino protecting group, R¹⁸ is carboxy or a protected carboxy group and wherein any optional substituent on the pyridyl ring is optionally protected; and wherein at least one protecting group is present; and thereinafter if necessary;
(i) forming a pharmaceutically acceptable salt,
(ii) esterifying to form an in vivo hydrolysable ester.

Protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

The compounds of the formula (V) are novel and form another aspect of the invention.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxy protecting group may be the residue of an ester-forming aliphatic or araliphatic alcohol or of an ester-forming silanol (the said alcohol or silanol preferably containing 1-20 carbon atoms).

Examples of carboxy protecting groups include straight or branched chain (1-12C)alkyl groups (eg isopropyl, t-butyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxycarbonyloxyethyl, 1-ethoxycarbonyloxyethyl); aryl lower alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); tri(lower alkyl)silyl lower alkyl groups (eg trimethylsilylethyl); diaryl(lower alkyl)silyl groups (eg t-butyldiphenylsilyl); and (2-6C)alkenyl groups (eg allyl and vinylethyl).

Methods particularly appropriate for the removal of carboxyl protecting groups include for example acid-, base-, metal- or enzymically-catalysed hydrolysis, for groups such as p-nitrobenzyloxycarbonyl, hydrogenation and for groups such as o-nitrobenzyloxycarbonyl, photolytically.

Examples of hydroxyl protecting groups include lower alkenyl groups (eg allyl); lower alkanoyl groups (eg acetyl); lower alkoxycarbonyl groups (eg t-butoxycarbonyl); lower alkenyloxycarbonyl groups (eg allyloxycarbonyl); aryl lower alkoxycarbonyl groups (eg benzoyloxycarbonyl, p-methoxybenzyloxycarbonyl, o-nitrobenzyloxycarbonyl, p-nitrobenzyloxycarbonyl); tri lower alkylsilyl (eg trimethylsilyl, t-butyldimethylsilyl); diaryl(lower alkyl)silyl groups (eg t-butyldiphenylsilyl) and aryl lower alkyl (eg benzyl) groups.

Examples of amino protecting groups include formyl, aralkyl groups (eg benzyl and substituted benzyl, eg p-methoxybenzyl, nitrobenzyl and 2,4-dimethoxybenzyl, and triphenylmethyl); di-p-anisylmethyl and furylmethyl groups; lower alkoxycarbonyl (eg t-butoxycarbonyl); lower alkenyloxycarbonyl (eg allyloxycarbonyl); aryl lower alkoxycarbonyl groups (eg benzyloxycarbonyl, p-methoxybenzyloxycarbonyl, o-nitrobenzyloxycarbonyl, p-nitrobenzyloxycarbonyl); trialkylsilyl (eg trimethylsilyl and t-butyldimethylsilyl); diaryl(lower alkyl)silyl group (eg t-butyldiphenylsilyl); alkylidene (eg methylidene); benzylidene and substituted benzylidene groups.

Methods appropriate for removal of hydroxy and amino protecting groups include, for example, acid-, base-, metal- or enzymically-catalysed hydrolysis, for groups such as p-nitrobenzyloxycarbonyl, hydrogenation and for groups such as o-nitrobenzyloxycarbonyl, photolytically.

In another aspect of the present invention the compounds of the formulae (I) and (V) may be prepared by
a) reacting compounds of the formulae (VI) and (VII): 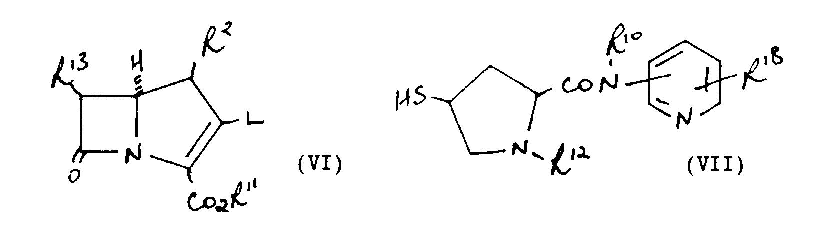 wherein R², R¹⁰, R¹¹, R¹², R¹³ and R¹⁸ are as hereinbefore defined, the pyridyl ring is optionally substituted as hereinbefore defined and L is a leaving group, or
b) cyclising a compound of the formula (VIII): 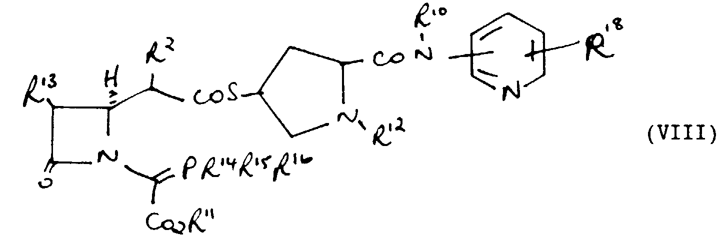 wherein R², R¹⁰,R¹¹, R¹², R¹³ and R¹⁸ are as hereinbefore defined, the pyridyl ring is optionally substituted as hereinbefore defined and R¹⁴, R¹⁵ and R¹⁶ are independently selected from C₁₋₆alkoxy, aryloxy, di-C₁₋₆alkylamino and diarylamino or any two of R¹⁴-R¹⁶ represent o-phenylenedioxy or one of R¹⁴ to R¹⁶ is C₁₋₄alkyl, alkyl, benzyl or phenyl and the other two values are independently selected from C₁₋₄alkyl, trifluoromethyl or phenyl wherein any phenyl group is optionally substituted with C₁₋₃ alkyl or C₁₋₃alkoxy; and wherein any functional group is optionally protected and thereinafter if necessary:
   (i) removing any protecting groups;
   (ii) forming a pharmaceutically acceptable salt;
   (iii) esterifying to form an in vivo hydrolysable ester.

Suitably in the compound of the formula (VI), L is the reactive ester of a hydroxy group such as a sulphonate (for example C₁₋₆alkanesulphonyloxy, trifluoromethanesulphonyloxy, benzenesulphonyloxy, toluenesulphonyloxy), a phosphoric ester (for example a diarylphosphoric ester such as diphenylphosphoric ester) or L is a halide (for example chloride). In an alternative L is a sulphoxide for example -SOCH=CH-NHCOCH₃ which may be readily displaced. Preferably L is diphenylphosphoric ester (-OP(O)(OPh)₂).

Compounds of the formula (VI) and their preparation are well known in the carbapenem literature, for example see EP-A-126587, EP-A-160391, EP-A-243686 and EP-A-343499.

The reaction between the compounds of the formulae (VI) and (VII) is typically performed in the presence of a base such as an organic amine for example di-isopropylethylamine or an inorganic base for example an alkali metal carbonate such as potassium carbonate. The reaction is conveniently performed at a temperature between -25°C and ambient. The reaction is generally performed in an organic solvent such as acetonitrile or dimethylformamide. The reaction is generally performed in a manner similar to that described in the literature for similar reactions.

The compounds of the formula (VII) are novel and form another aspect of the present invention.

The compounds of the formula (VII) may be prepared by the deprotection of a compound of the formula (IX): 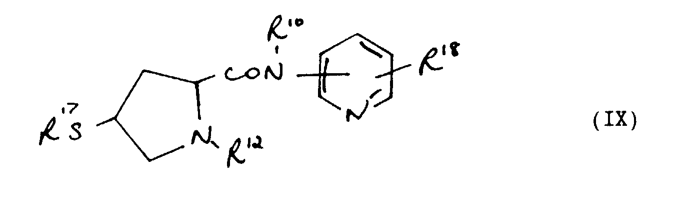 wherein R¹⁰, R¹² and R¹⁸ are as hereinbefore defined, the pyridyl ring is optionally substituted as hereinbefore defined and R¹⁷ is a protecting group, for example C₁₋₆alkanoyl or C₁₋₆alkoxycarbonyl. Preferred values for R¹⁷ are acetyl and t-butoxycarbonyl. The compounds of the formula (IX) can be converted to the compounds of the formula (VII) by standard methods of deprotection, for example acetyl groups can be removed by basic hydrolysis in aqueous alkanol, alkenol or a cyclic ether for example methanol, allyl alcohol or tetrahydrofuran.

The compounds of the formula (IX) are novel and form another aspect of the present invention.

The compounds of the formula (IX) may be prepared by the reaction of an activated derivative of a compound of the formula (X), which may be formed in situ, with a compound of the formula (XI): 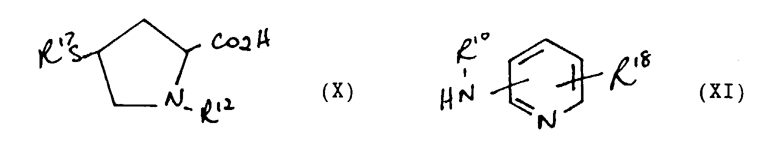 wherein R¹⁰, R¹², R¹⁷ and R¹⁸ are as hereinbefore defined and the pyridyl ring is optionally substituted as hereinbefore defined. Activated derivatives of the compound of the formula (X) include acid halides, anhydrides and 'activated' esters such as 1H-benzol-1,2,3-triazol-1-yl, pentafluorophenyl and 2,4,5-trichlorophenyl esters or the benzimidazol-2-yl ester of the thiocarboxylic acid corresponding to (X). The reaction of an activated derivative of a compound of the formula (X) and a compound of the formula (XI) is performed under standard methods, for example in dichloromethane, at 0°C in the presence of trimethylsilylchloride and diisopropylethylamine.

The compounds of the formulae (X) and (XI) are prepared by standard methods known to the skilled chemist such as the methods of the Examples hereinafter, the methods described in EP-A-126587 or by methods analogous or similar thereto.

Suitably, in the compounds of the formula (VIII), R¹⁴, R¹⁵ and R¹⁶ are independently selected from C₁₋₆ alkoxy such as methoxy, ethoxy, isopropoxy, n-propoxy or n-butoxy; aryloxy such as optionally phenoxy; di-C₁₋₆alkylamino such as dimethylamino or diethylamino; diarylamino such as diphenylamino or any two of R¹⁴ -R¹⁶ represent o-phenylenedioxy. Preferably each of R¹⁴-R¹⁶ have the same value and are C₁₋₆ alkoxy for example methoxy, ethoxy, isopropoxy or n-butoxy or are phenoxy.

The compounds of the formula (VIII) are cyclized under conventional conditions known in the art to form compounds of the formula (V). Typical conditions are heating in a substantially inert organic solvent such as toluene, xylene or ethyl acetate at temperatures in the region 60-150°C. Typically the reaction is performed in an atmosphere of nitrogen and is carried out in the presence of a radical scavenger for example hydroquinone.

The compounds of the formula (VIII) may be formed and cyclized in situ. The compounds of the formula (VIII) may conveniently be prepared by reacting compounds of the formulae (XII) and (XIII):

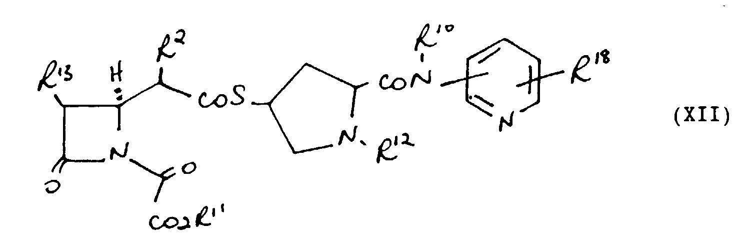

PR¹⁴R¹⁵R¹⁶ (XIII)

wherein R², R¹⁰, R¹¹-R¹⁶, and R¹⁸ are as hereinbefore defined and the pyridyl ring is optionally substituted as hereinbefore defined. Suitably the compound of the formula (XIII) is a phosphite or is the functional equivalent of such a compound.

The reaction between the compounds of the formulae (XII) and (XIII) is conveniently performed in an organic solvent such as toluene, xylene, ethyl acetate, chloroform, dichloromethane, acetonitrile or dimethylformamide. Typically the reaction is carried out at an elevated temperature for example 60-150°C.

The compounds of the formula (XII) may be prepared by a number of methods known in the art. For example the compounds of the formula (XII) may be prepared by the acylation of a compound of the formula (XIV): 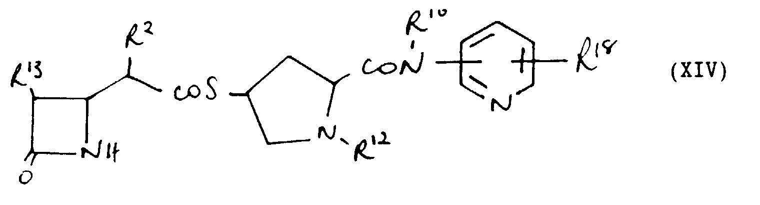 wherein R², R¹⁰, R¹², R¹³, and R¹⁸ are as hereinbefore defined and the pyridyl ring is optionally substituted as hereinbefore defined with a compound of the formula (XV):

Cl-CO-COOR¹¹ (XV)

wherein R¹¹ is as hereinbefore defined.

The compounds of the formula (XIV) may be prepared by reacting compounds of the formulae (XVI) and (VII): 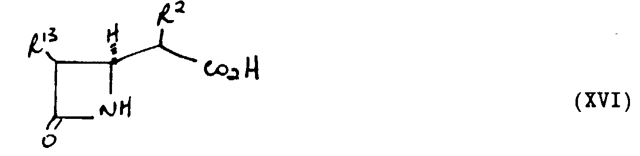 wherein R² and R¹³ are as hereinbefore defined. The compounds of the formula (XVI) are known in the art and may be reacted with the compounds of the formula (VII) under conventional acylation methods known in the art.

Compounds of the formulae (VII), (XII) and (XIV) are novel and, as such, form another aspect of this invention.

The following biological test methods, data and Examples serve to illustrate the present invention.

### Antibacterial Activity

The pharmaceutically acceptable carbapenem compounds of the present invention are useful antibacterial agents having a broad spectrum of activity in vitro against standard laboratory microorganisms, both Gram-negative and Gram-positive, which are used to screen for activity against pathogenic bacteria. The antibacterial spectrum and potency of a particular compound may be determined in a standard test system. In particular the carbapenems of the present invention show good stability to beta-lactamases and in general particularly good pharmacokinetics, especially as regards half life. In general compounds show significant improvement over imipenem.

The antibacterial properties of the compounds of the invention may also be demonstrated in vivo in conventional tests.

Carbapenem compounds have generally been found to be relatively non-toxic to warm-blooded animals, and this generalisation holds true for the compounds of the present invention. Compounds representative of the present invention were administered to mice at doses in excess of those required to afford protection against bacterial infections, and no overt toxic symptoms or side effects attributable to the administered compounds were noted.

The following results were obtained for representative compounds on a standard in vitro test system using Diagnostic Sensitivity Test. The antibacterial activity is described in terms of the minimum inhibitory concentration (MIC) determined by the agar-dilution technique with an inoculum size of 10⁴ CFU/spot.

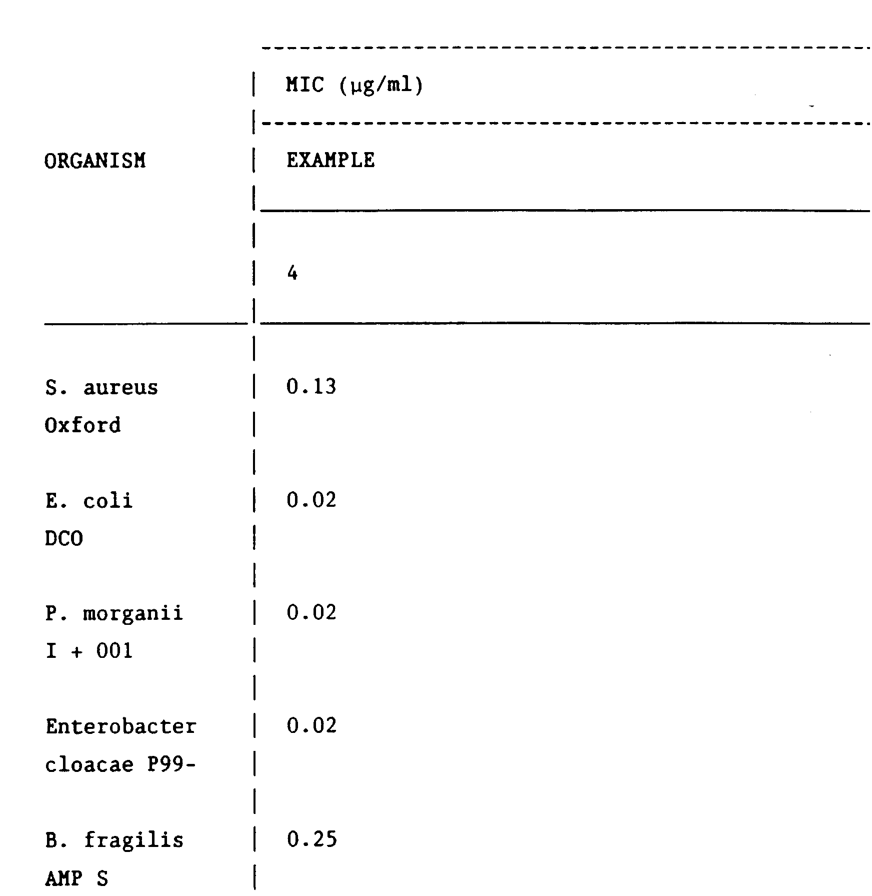

In the following examples, which are representative of the scope:
(a) NMR spectra were taken at 200MHz or 400MHz in DMSO-d₆/CD₃COOD unless otherwise stated;
(b) allyloxy means the propen-1-yloxy group -OCH₂CH=CH₂;
(c) DMF means dimethylformamide;
(d) DMSO means dimethylsulphoxide;
(e) evaporation of solvents was carried out under reduced pressure;
(f) HPLC means high pressure liquid chromatography;
(g) temperatures are given in degrees centigrade.

### Example 1

(1R,5S,6S,8R,2'S,4'S)-2-(2-(4-Carboxy-2-pyridylcarbamoyl)pyrrolidin-4-ylthio)-6-(1-hydroxyethyl)-1-methylcarbapenem-3-carboxylic acid

A solution of 4-nitrobenzyl(1R,5S,6S,8R,2'S,4'S)-2-(1-(4-nitrobenzyloxycarbamoyl)-2-(4-carboxy-2-pyridylcarbamoyl)pyrrolidin-4-ylthio)-6-(1-hydroxyethyl)-1-methylcarbapenem-3-carboxylate (695 mg, 0.88 mmol) in a mixture of methanol/ethyl acetate (1/4) (20 ml), water (20 ml) and KHCO₃ (200 mg, 2 mmol) was hydrogenated over Pd/carbon (10%), (400 mg). The reaction was followed by HPLC. The residue is purified by subjecting to preparative HPLC (Nucleosil C-18) after filtration of the solid and concentration, to give the title compound (76 mg, 16%).
NMR: δ 1.16 (m, 6H); 1.75 (m, 1H); 2.6-2.74 (m, 2H); 3.2 (dd, 1H) 3.3-3.5 (m, 2H); 3.63 (m, 1H); 3.96 (m, 2H); 4.15 (m, 1H); 7.5 (d, 1H); 8.37 (d, 1H); 8.53 (s, 1H).

The starting material was prepared as follows:

(2S,4S)-1-(4-Nitrobenzyloxycarbonyl)-4-acetylthio-carboxypyrrolidine (1.5 g, 4 mmol) was treated at ambient temperature, for 5 hours, with thionyl chloride (12 ml) and a catalytic amount of DMF (15 mg). The solvent was evaporated, the residue taken up in CH₂Cl₂, evaporated, dried under reduced pressure for 1 hour and solubilized in CH₂Cl₂ (10 ml). This solution was added to a cold (0°C) solution of 2-amino-4-carboxypyridine (560 mg, 4 mmol) diisopropylethylamine (2.12 ml, 12 mmol) and trimethylsilylchloride (1 ml, 12 mmol) in dry CH₂Cl₂. After 12 hours at ambient temperature, the solvent was evaporated, the residue purified by subjecting to chromatography on HP20SS resin, (eluant: CH₃C N/H₂O) to give (2S,4S)-1-(4-nitrobenzyloxycarbonyl)-2-(4-carboxy-2-pyridylcarbamoyl)-pyrrolidin-4-ylthioacetate (650 mg, 32.5%).
NMR: δ 1.9 (m, 1H); 2.32 (s, 3H); 2.83 (m, 1H); 3.36 (m, 1H); 4.0 (m, 2H); 4.64 (s, 1H); 4.9-5.35 (m, 2H); 7.4-7.7 (m, 3H); 7.92 (s, 1H); 8.2 (s, 1H); 8.47 (m, 2H).

(2S,4S)-1-(4-Nitrobenzyloxycarbonyl)-2-(4-carboxy-2-pyridyl-carbamoyl)pyrrolidin-4-ylthioacetate (488 mg, 1 mmol) in methanol (20 ml) was treated with a 1M aqueous solution of NaOH (2.5 ml, 2.5 mmol) under argon, at 0°C. The reaction was followed by HPLC. After 1 hour the reaction mixture was acidified (at pH 6.5, 0°C with 6M HCl), the solvent evaporated and dried under reduced pressure for 1 hour. The resulting crude thiol was solubilized in DMF (5 ml) and added to a cold (0°C) solution of 4-nitrobenzyl (1R,5R,6S,8R)-6-(1-hydroxy)-1-methyl-2-diphenylphosphoryloxycarbapenem-3-carboxylate (499 mg, 0.84 mmol) in DMF (5 ml). This solution was treated successively with diisopropylethylamine (350 µl, 1 mmol) tri-n-butylphosphine (250 µl, 1 mmol) and water (20 µl, 1 mmol), and stirred at ambient temperature overnight. The crude reaction mixture was purified by subjecting to chromatography on a HP20SS column, to give 4-nitrobenzyl (1R,5R,6S,8R,2'S,4'S)-2-(1-(4-nitrobenzyloxycarbonyl)-2-(4-carboxy-2-pyridylcarbamoyl)pyrrolidin-4-ylthio)-6-(1-hydroxyethyl)-1-methylcarbapenem-3-carboxylate (695 mg, 88%) (eluting with CH₃CN/H₂O gradient of CH₃CN.)
NMR: δ 1.4 (m, 6H); 1.9 (m, 1H); 2.83 (m, 1H); 3.3-3.5 (m, 2H); 3.6 (m, 1H); 3.9-4.1 (m, 2H); 4.1-4.3 (m, 2H); 3.6 (m, 1H); 3.6 (m, 1H); 5.0-5.5 (m, 4H); 7.4-7.8 (m, 5H); 7.9 (d, 1H); 8.2 (m, 3H); 8.4-8.6 (m, 2H).

### Example 2

### (1R,5S,6S,8R,2'S,4'S)-2-(2-(3-Carboxy-5-pyridylcarbamoyl)-pyrrolidin-4-ylthio)-6-(1-hydroxyethyl)-1-methylcarbapenem-3-carboxylic acid.

The title compound was prepared from the corresponding 4-nitrobenzyl protected compound using a similar method to that of example 1.
NMR: δ 0.85 (m, 6H); 1.28 (m, 4H); 1.3-1.5 (m, 4H); 2.63 (m, 1H); 2.8 (m, 1H); 3.2 (dd, 1H); 3.4 (m, 2H); 3.65 (m, 1H); 3.95 (m, 2H); 4.15 (m, 1H); 8.58 (m, 1H); 8.76 (s, 1H); 8.93 (m, 1H).

(2S,4S)-1-(4-Nitrobenzyloxycarbonyl)-2-(3-carboxy-5-pyridyl carbamoyl)pyrrolidin-4-ylthioacetate was prepared using a similar method to that of example 1, except using 5-amino-3-carboxypyridine acid in place of 2-amino-4-carboxypyridine.
NMR: δ 1.9 (m, 1H); 2.32 (s, 3H); 2.83 (m, 1H); 3.36 (m, 1H); 4.0 (m, 2H); 4.5 (m, 1H); 5.0-5.35 (m, 2H); 7.5 (d, 1H); 7.7 (d, 1H); 7.95 (d, 1H); 8.25 (d, 1H); 8.5-9.0 (m, 3H).

Allyl (1R,5R,6S,8R,2'S,4'S)-2-(1-(4-nitrobenzyloxycarbonyl)-2-(3-carboxy-5-pyridylcarbamoyl)pyrrolidin-4-ylthio)-6-(1-hydroxyethyl) -1-methylcarbapenem-3-carboxylate was prepared using a similar method to that of example 1, except using allyl (1R,5R,6S,8R)-6-(1-hydroxyethyl)-1-methyl-2-diphenylphosphorylcarbapenem-3-carboxylate as the carbapenem precursor, and the thioacetate product of the previous step in place of (2S,4S)-1-(4-nitrobenzyloxycarbonyl)-2-(4-carboxy-2-pyridylcarbamoylpyrrolidin-4-ylthioacetate.
NMR: δ 1.27 (m, 6H); 1.95 (m, 1H); 2.8 (m, 1H); 3.26 (dd, 1H); 3.38 (m, 1H); 3.55 (m, 1H); 3.98 (m, 2H); 4.1 (m, 1H); 4.25 (m, 1H); 4.4-4.75 (m, 3H); 5.5-5.45 (m, 4H); 5.88 (m, 1H).

Allyl (1R,5R,6S,8R,2'S,4'S) 2-(1-(4-nitrobenzyloxycarbonyl)-2-(3-carboxy-5-pyridylcarbamoyl)pyrrolidin-4-ylthio)-6-(1-hydroxyethyl)-1-methylcarbapenem-3-carboxylate (450 mg, 0.65 mmol) in THF (25 ml) was treated at ambient temperature with triphenylphosphine (17 mg, 0.065 mmol), potassium hexanoate 0.46 M in ethyl acetate (3.4 ml, 1.55 mmol) and Pd(PPh₃)₄ (25 mg, 0.02 mmol) for 1 hour. The mixture was diluted with ethyl acetate (25 ml), the solid filtered, washed with ethyl acetate and dried (395 mg, 83.5%). This compound was used in the following step without further purification.

### Example 3

### (1R,5S,6S,8R,2'S,4'S)-2-(2-(3-Carboxy-2-pyridylcarbamoyl) pyrrolidin-4-ylthio)-6-(1-hydroxyethyl)-1-methylcarbapenem-3-carboxylic acid.

The title compound was prepared from the corresponding 4-nitrobenzyl protected compound, using a similar method to that of example 1.
NHR: δ 1.15 (2d, 6H), 1.74 (m, 1H); 2.7-2.95 (m, 2H); 3.2 (dd, 1H); 3.39 (m, 1H); 3.51 (m, 1H); 3.71 (m, 1H); 3.96 (q, 1H); 4.15 (dd, 1H); 4.39 (m, 1H); 7.11 (m, 1H); 8.23-8.38 (m, 2H).

The starting material was prepared as follows:

(2S,4S) 1-(4-Nitrobenzyloxycarbonyl)-2-(3-carboxypyridyl-carbamoyl)pyrrolidin-4-ylthioacetate was prepared using a similar method of that of example 1, except using 2-amino-3-carboxypyridine in place of 2-amino-4-carboxypyridine.
NMR: δ 2.05 (m, 1H); 2.3 (s, 3H); 2.85 (m, 1H); 3.35 (m, 1H); 3.8-4.25 (m, 2H); 4.74 (dd, 1H); 5.16 (m, 2H); 7.18 (dd, 1H); 7.5 (d, 2H); 8.04 (d, 2H); 8.23 (dd, 1H); 8.46 (dd, 1H).

Allyl (1R,5R,6S,8R,2'S,4'S) 2-(1-(4-nitrobenzyloxycarbonyl)-2-(3-carboxy-2-pyridylcarbamoylpyrrolidin-4-ylthio)-6-(1-hydroxyethyl)-1-methylcarbapenem-3-carboxylate (diisopropylethylamine salt) was prepared using a similar method to that of example 2 from the thioacetate product of the previous step.
NMR: δ 1.06-1.35 (m, 21H); 2.0 (m, 1H); 2.88 (m, 1H); 3.12 (q, 2H; 3.25 (dd, 1H); 3.35 (m, 1H); 3.42-3.68 (m, 3H); 3.9-4.05 (m, 2H); 4.1-4.3 (m, 2H); 4.46-4.8 (m, 3H); 5.0-5.42 (m, 4H); 5.85 (m, 1H); 7.15 (dd, 1H); 7.45 (d, 1H); 7.68 (d, 1H); 7.93 (d, 1H); 8.15-8.31 (m, 2H); 8.4 (m, 1H).

(1R,5R,6S,8R,2'S,4'S) 2-(1-(4-Nitrobenzyloxycarbonyl)-2-(3-carboxy-2-pyridylcarbamoyl)pyrrolidin-4-ylthio)-6-(1-hydroxyethyl)-1-methylcarbapenem-3-carboxylic acid was prepared from the allyl protected compound of the last step using a similar method to that of example 2.

### Example 4

### (1R,5R,6S,8R,2'S,4'S)-2-(2-(5-Carboxy-2-pyridylcarbamoyl)pyrrolidin-4-ylthio)-6-(1-hydroxyethyl)-1-methylcarbapenem-3-carboxylic acid.

The title compound prepared from the corresponding 4-nitrobenzyl protected compound, using a similar method to that of example 1.
NMR: δ 1.15 (2d, 6H); 1.75 (m, 1H); 2.55-2.79 (m, 2H); 3.18 (dd, 1H); 3.3-3.5 (m, 2H); 3.63 (m, 1H); 3.9-4.07 (m, 2H); 4.14 (dd, 1H): 8.17 (d, 1H); 8.28 (dd, 1H); 8.82 (m, 1H).

The starting material was prepared as follows:

(2S,4S)-1-(4-Nitrobenzyloxycarbonyl)-2-(5-carboxy-2-pyridylcarbamoyl)pyrrolidin-4-ylthioacetate was prepared using a similar method to that of example 1, except using 2-amino-5-carboxypyridine in place of 2-amino-4-carboxypyridine.
NMR: δ 2.05 (m, 1H); 2.3 (s, 3H); 2.85 (m, 1H); 3.37 (m, 1H); 3.8-4.25 (m, 2H); 4.66 (m, 1H); 5.18 (m, 2H); 7.52 (d, 2H); 7.88-8.33 (m, 4H); 8.78 (m, 1H).

Allyl (1R,5R,6S,8R,2'S,4'S) 2-(1-nitrobenzyloxycarbonyl)-2-(5-carboxy-2-pyridylcarbamoyl)pyrrolidin-4-ylthio)-6-(1-hydroxyethyl)-1-methylcarbapenem-3-carboxylate was prepared from the thioacetate product of the previous step using a similar method to that of example 2.
NMR: δ 1.15 (2d, 6H); 1.9 (m, 1H); 2.83 (m, 1H); 3.22-3.44 (m, 2H); 3.58 (q, 1H); 3.86-4.06 (m, 2H); 4.1-4.3 (m, 2H); 4.55-4.8 (m, 3H); 5.16-5.48 (m, 4H); 5.92 (m, 1H); 7.46 (d, 1H); 7.67 (d, 1H); 7.92 (d, 1H); 8.08-8.32 (m, 3H); 8.83 (d, 1H).

(1R,5R,6S,2'S,4'S) 2-(1-(4-Nitrobenzyloxycarbonyl)-2-(5-carboxy-2-pyridylcarbamoyl)pyrrolidin-4-ylthio)-6-(1-hydroxyethyl)-1-methylcarbapenem-3-carboxylic acid was prepared from the product of the previous step using a similar method to that of example 2.

### Example 5

### (1R,5S,6S,8R,2'S,4'S)-2-(2-(2-Carboxy-4-pyridylcarbamoyl)pyrrolidin-4-ylthio)-6-(1-hydroxyethyl)-1-methylcarbapenem-3-carboxylic acid (2K+).

A solution of(1R,5S,6S,2'S,4'S)-2-(1-(4-nitrobenzyloxycarbonyl)-2-(2-carboxy-4-pyridylcarbamoyl)pyrrolidin-4-ylthio)-6-(1-hydroxyethyl)-1-methylcarbapenem-3-carboxylic acid (2K⁺) (0.4 g, 0.55 mmol) in water (15 ml) was hydrogenated at atmospheric pressure in the presence of 10% Pd/C. (0.2 g) and potassium bicarbonate (55 mg, 0.55 mmol). The progress of the reaction was followed by analytical HPLC. The catalyst was removed by filtration, the filtrate purified by preparative HPLC on C₁₈ Nucleosil, eluting with water. The product was recovered and freeze dried (0.1 g, 33%).
NMR (DMSO + CD₃COOD): δ 1.16 (m, 6H); 1.76 (m, 1H); 2.62 (m, 1H; 2.77 (m, 1H); 3.19 (dd, 1H); 3.37 (m, 2H); 3.64 (m, 1H); 3.96 (m, 2H); 4.15 (dd, 1H); 7.88 (dd, 1H); 8.34 (d, 1H); 8.51 (d, 1H).

The starting material was prepared as follows:

A solution of 2-carboxy-4-nitropyridine N-oxide (0.5 g, 27 mmol) (E. Prafft et al., J. Prakt. Chem. 1961, 13, 58) in acetic acid (20 ml) was hydrogenated under pressure (70 psi) in the presence of Pd/C. (10%) (0.25 g) at ambient temperature for 2 hours. The catalyst was removed by filtration, and the filtrate evaporated to give 2-carboxy-4-aminopyridine as an off-white solid which was dried in a dessicator (300 mg, 80%).
NMR (D₂O): δ 6.75 (d, 1H); 7.17 (s, 1H); 8.11 (d, 1H).

(2S,4S)-1-(4-Nitrobenzyloxycarbonyl)-4-acetylthio-2-carboxypyrrolidine (1.5 g, 4 mmol) in CH₂Cl₂ (10 ml) was treated at ambient temperature, for 12 hours, with thionyl chloride (1.4 ml, 19 mmol) and a catalytic amount of DMF (20 µl). The solution was then evaporated, and the residue dried under vacuum for two hours, solubilized in CH₂Cl₂ (10 ml) and added to a solution of 2-carboxy-4-aminopyridine (0.887 g, 4.5 mmol), diisopropylethylamine (35 ml, 20 mmol) and trimethylsilylchloride (3 ml, 25 mmol) in CH₂Cl₂ (20 ml), at 0°C. The reaction was monitored by HPLC. After 12 hours at ambient temperature, the mixture was evaporated and the residue purified by chromatography on a HP20SS column, eluting with CH₃CN/H₂O/AcOH (1:1:1/100). The required fractions were collected and freeze dried to give (2S,4S)-1-(4-nitrobenzyloxycarbonyl)-2-(2-carboxy-4-pyridylcarbamoyl)pyrrolidin-4-ylthioacetate (1.06 g, 53%).
NMR (DHSO + CD₃COOD): δ 2.0 (m, 1H); 2.33 (s, 3H); 2.83 (m, 1H), 3.4 (m, 1H); 4.1 (m, 2H); 4.6 (m, 1H); 5.2 (m, 2H); 7.5 (m, 1H); 7.67 (m, 1H); 8.02 (m, 1H); 8.15 (m, 2H); 8.5-8.8 (m, 2H).

(2S,4S)-1-(4-Nitrobenzyloxycarbonyl)-2-(2-carboxy-4-pyridyl-carbamoyl)pyrrolidin-4-ylthioacetate (0.448 g, 1 mmol) in MeOH (20 ml) was treated with 1N NaOH (2.5 ml, 2.5 mmol), added drop by drop, at ambient temperature. After 30 minutes the mixture was acidified with 6N HCl, at 0°C, and the solvent evaporated. The residue was solublized in DMF (5 ml) and added to a solution of allyl (1R,5R,6S,8R)-6-(1-hydroxyethyl)-1-methyl-2-diphenylphosphoryloxycarbapenem-3-carboxylate (0.499 g, 1 mmol), in DMF (5 ml), in the presence of N,N'-diisopropylethylamine (0.35 ml, 2 mmol), water (18 µl, 1 mmol) and n-tributylphosphine (0.25 ml, 1 mmol). The mixture was left for 12 hours at ambient temperature. The crude reaction mixture was poured onto a HP20SS column, and eluted with CH₃CN/H₂O (gradient of CH₃CN) to give allyl(1R,5R,6S,8R,2'S,4'S)-2-(1-(4-nitrobenzyloxycarbonyl)-2-(2-carboxy-4-pyridylcarbamoyl)pyrrolidin-4-ylthio)-6-(1-hydroxyethyl)-1-methylcarbapenem-3-carboxylate (0.4 g, 57.5%).
NMR: (DMSO + CD₃COOD): δ 1.26 (m, 6H); 1.95 (m, 1H); 2.82 (m, 1H); 3.27 (dd, 1H); 3.4 (m, 1H); 3.6 (m, 1H); 3.98 (m, 2H); 4.13 (m, 1H); 4.25 (m, 1H); 4.44-4.7 (m, 3H); 5.05-5.42 (m, 4H); 5.88 (m, 1H); 7.47 (m, 1H); 7.67 (m, 1H); 7.86-7.96 (m, 2H); 8.23-8.30 (m, 2H); 8.54 (m, 1H).

Allyl(1R,5R,6S,8R,2'S,4'S)-2-(1-(4-nitrobenzyloxycarbonyl)-2-(2-carboxy-4-pyridylcarbamoyl)pyrrolidin-4-ylthio)-6-(1-hydroxyethyl)-1-methylcarbapenem-3-carboxylate (0.4 g, 0.575 mmol), in THF (20 ml), was treated successively with PPh₃ (15 mg, 0.05 mmol), potassium hexanoate in ethyl acetate (3.6 ml, 1.43 mmol, 0.47M) and Pd(PPh₃)₄ (20 mg, 0.017 mmol). The reaction was followed by HPLC. After 15 minutes, the reaction mixture was diluted with ethyl acetate (20 ml) and the precipitate recovered by filtration and dried to give (1R,5R,6S,8R,2'S,4'S)-2-(1-(4-nitrobenzyloxycarbonyl-2-(2-carboxy-4-pyridylcarbamoyl)pyrrolidin-4-ylthio)-6-(1-hydroxyethyl)-1-methyl-carbapenem-3-carboxylic acid (2K⁺) (0.4 g, 95%).
NMR (DMSO + CD₃COOD): δ 1.15 (m, 6H); 1.95 (m, 1H); 2.8 (m, 1H); 3.2 (dd, 1H); 3.4 (m, 2H); 3.8-4.2 (m, 4H); 4.5 (m, 1H); 5.0-5.3 (m, 2H); 7.47 (m, 1H); 7.67 (m, 1H); 7.75-8.00 (m, 2H); 8.25 (m, 2H); 8.5 (m, 1H).

### Example 6

### (1R,5S,6S,8R,2'S,4'S)-2-(2-Carboxy-6-pyridylcarbamoyl)pyrrolidin-4-ylthio)-6-(1-hydroxyethyl)-1-methylcarbapenem-3-carboxylic acid (2K⁺).

The deprotection of(1R,5S,6S,2'S,4'S)-2-(1-(4-nitrobenzyloxycarbonyl)-2-(2-carboxy-6-pyridylcarbamoyl)pyrrolidin-4-ylthio)-6-(1-hydroxyethyl)-1-methylcarbapenem-3-carboxylic acid (2K⁺) was carried out using a similar method to that described in Example 5.
NMR (DMSO-d₆ + CD₃COOD) δ 1.15 (m, 6H); 1.85 (m, 1H); 2.78 (m, 1H); 2.93 (m, 1H); 3.19 (dd, 1H); 3.37 (dt, 1H), 3.56 (m, 1H); 3.76 (m, 1H); 3.96 (dq; 1H); 4.18 (m, 2H); 7.76 (d, 1H); 7.92 (m, 1H); 8.24 (d, 1H).

The starting material was prepared as follows:

A solution of 2-carboxy-6-aminopyridine (0.4 g, 2.89 mmol) in DHF (4 ml) was treated successively with allylbromide (0.37 ml, 4.33 mmol) and potassium carbonate (0.48 g, 3.47 mmol). The mixture was stirred for one hour at ambient temperature and kept for two hours at 60°C. The reaction mixture was then cooled, poured on ice, extracted with ethyl acetate, washed with water and dried over MgSO₄. The required product was obtained by silica gel chromatography, eluting with petroleum ether/ether (1:1), to give 2-allyloxycarbonyl-6-aminopyridine (295 mg, 57%).
NMR (CDCl₃): δ 4.79 (m, 2H); 4.87 (m, 2H); 5.29 (m, 1H); 5.42 (m, 1H); 6.05 (m, 1H); 6.67 (m, 1H); 7.5 (m, 2H).

(2S,4S)-1-(4-Nitrobenzyloxycarbonyl)-4-acetylthio-2-carboxy-pyrrolidine (0.62 g, 1.69 mmol) in dry CH₂Cl₂ (5 ml) was treated with thionyl chloride (0.6 ml) and a catalytic amount of DMF (10 µl) for 12 hours, at ambient temperature. The solution was then evaporated, and the residue dried under vacuum for two hours. The residue was solubilized in CH₂Cl₂ (4 ml) and added to a solution of 2-allyloxy-carbonyl-6-aminopyridine(0.275 g, 1.55 mmol) and diisopropylethylamine (0.27 ml, 1.55 mmol) in CH₂Cl₂ (4 ml), at 0°C. The mixture was stirred at ambient temperature for two hours, the solvent evaporated, and the residue purified by silica gel chromatography, eluting with CH₂Cl₂/ether (9:1), to give (2S,4S)-1-(4-nitrobenzyloxycarbonyl)-2-(2-allyloxycarbonyl-6-pyridylcarbamoyl)pyrrolidin-4-ylthioacetate (0.69 g, 85%).
NMR (CDCl₃): δ 2.28 (m, 1H); 2.31 (s, 3H); 2.76 (m, 1H); 3.45 (m, 1H); 4.01 (m, 1H); 4.17 (m, 1H); 4.47 (m, 1H); 4.88 (m, 2H); 5.37 (m, 4H); 6.04 (m, 1H); 7.47 (m, 1H); 7.85 (m, 2H); 7.9-8.45 (m, 4H).

(2S,4S)-1-(4-Nitrobenzyloxycarbonyl)-2-(2-allyloxycarbonyl-6-pyridylcarbamoyl)pyrrolidin-4-ylthioacetate (0.69 g, 1.3 mmol) in a mixture of EtOH (7.5 ml) and CH₂Cl₂ (2 ml) was treated with a solution of methylamine in EtOH (2.6 mmol) for 1 hour, at 0°C. The mixture was concentrated, the residue solublized in CH₃CN (10 ml) and added to a solution of allyl (1R,5R,65,8R)-6-(1-hydroxyethyl)-1-methyl-2-diphenylphosphoryloxycarbapenem-3-carboxylate (0.65 g, 1.3 mmol) in acetonitrile (5 ml). This mixture was then treated successively with N,N'-diisopropylethylamine (0.45 ml, 2.6 mmol), water (25 µl, 1.3 mmol) and n-tributylphosphine (0.32 ml, 1.3 mmol). The mixture was stirred for 3 hours at ambient temperature, evaporated to dryness and the residue purified by silica gel chromatography, eluting with EtOAc, to give allyl (1R,5R,6S,8R,2'S,4'S)-2-(1-(4-nitrobenzyloxycarbonyl)-2-(2-allyloxycarbonyl-6-pyridylcarbamoyl)pyrrolidin-4-ylthio)-6-(1-hydroxyethyl)-1-methylcarbapenem-3-carboxylate (0.5 g, 52%).
NMR (DMSO-d₆): δ 1.15 (m, 6H); 1.86 (m, 1H); 2.81 (m, 1H); 3.3 (m, 2H); 3.58 (m, 1H); 3.94 (m, 2H); 4.1-4.3 (m, 2H); 4.55-4.9 (m, 5H); 4.95-5.5 (m, 6H); 5.90 (m, 1H); 6.05 (m, 1H); 7.46-7.7 (m, 2H); 7.75-8.1 (m, 3H); 8.2-8.35 (m, 2H).

The allyl group was removed from allyl (1R,5R,6S,8R,2'S,4'S)-2-(1-(4-nitrobenzyloxycarbonyl)-2-(2-allyloxycarbonyl-6-pyridylcarbamoyl)pyrrolidin-4-ylthio)-6-(hydroxyethyl)-1-methylcarbapenem-3-carboxylate using a similar method to that described in Example 5, to give (1R,5R,6S,8R,2'S,4'S)-2-(1-(4-nitrobenzyloxycarbonyl)-2-(2-carboxy-6-pyridylcarbamoyl)pyrrolidin-4-ylthio)-6-(1-hydroxyethyl)-1-methylcarbapenem-3-carboxylic acid (2K⁺).

### Example 7

### (1R,5S,6S,8R,2'R,4'S)-2-(2-Carboxy-6-pyridylcarbamoyl)-pyrrolidin-4-ylthio)-6-(1-hydroxyethyl)-1-methylcarbarpenem-3-carboxylic acid (2K⁺).

The deprotection of (1R,5S,6S,8R,2'R,4'S)-2-(1-(4-nitrobenzyloxycarbonyl)-2-(2-carboxy-6-pyridylcarbamoyl)pyrrolidin-4-ylthio)-6-(1-hydroxyethyl)-1-methylcarbapenem-3-carboxylic acid (2K⁺) was carried out using a similar method to that described in Example 5, to give the title compound.
NMR (DMSO-d₆): δ 1.15 (m, 6H); 2.08 (m, 1H); 2.35 (m, 1H); 2.95 (m, 1H); 3.17 (dd, 1H); 3.34 (m, 2H); 3.76 (m, 1H); 3.95 (m, 1H); 4.14 (m, 2H); 7.73 (m, 1H); 7.91 (m, 1H); 8.24 (m, 1H).

The starting material was prepared as follows:

(2R,4S)-1-(4-Nitrobenzyloxycarbonyl)-4-acetylthio-2-carboxypyrrolidine (1.5 g, 4 mmol) in dry CH₂Cl₂ was treated with thionyl chloride (1.4 ml, 19 mmol) and a catalytic amount of DMF (20 µl) for 12 hours at ambient temperature. The solution was evaporated, and the residue dried under vacuum for 2 hours. The residue was solubilized in CH₂Cl₂ (10 ml) and added to a solution of 2-carboxy-6-aminopyridine (G. Ferrari et al., Farmaco (pavia) Ed. Sci. 1959, 14, 594. CA 53, 7162b) (560 mg, 4 mmol) in CH₂Cl₂ (20 ml), diisopropylethylamine (3.5 ml, 20 mmol) and trimethylsilylchloride (3 ml, 24 mmol), at 0°C. The mixture was stirred at ambient temperature for 4 hours, the solvent evaporated and the residue purified by HP20SS chromatography, eluanting with CH₃CN/H₂O/AcOH (1:1:1/100) to give (2R,4S)-1-(4-nitrobenzyloxycarbonyl)-2-(2-carboxy-6-pyridylcarbamoyl)-pyrrolidin-4-ylthioacetate (1g, 50%).

(2R,4S)-1-(4-Nitrobenzyloxycarbonyl)-2-(2-carboxy-6-pyridylcarbamoyl)pyrrolidin-4-ylthioacetate was reacted with allyl (1R,5R,6S,8R)-6-(1-hydroxyethyl)-1-methyl-2-diphenylphosphoryloxycarbapenem-3-carboxylate using a similar method to that described in Example 5, to give allyl (1R,5R,6S,8R,2'R,4'S)-2-(1-(4-nitrobenzyloxycarbonyl)-2-(2-carboxy-6-pyridylcarbamoyl)pyrrolidin-4-ylthio)-6-(1-hydroxyethyl)-1-methylcarbapenem-3-carboxylate.

The allyl protecting group was removed from allyl (1R,5R,6S,8R,2'R,4'S)-2-(1-(4-nitrobenzyloxycarbonyl)-2-(2-carboxy-6-pyridylcarbamoyl)pyrrolidin-4-ylthio)-6-(1-hydroxyethyl)-1-methylcarbapenem-3-carboxylate using a similar method to that described in Example 5, to give (1R,5R,6S,8R,2'R,4'S)-2-(1-(4-nitrobenzyloxycarbonyl)-2-(2-carboxy-6-pyridylcarbamoyl)pyrrolidin-4-ylthio)-6-(1-hydroxyethyl)-1-methylcarbapenem-3-carboxylic acid (2K⁺).

## Claims

1. A compound of the formula (I): 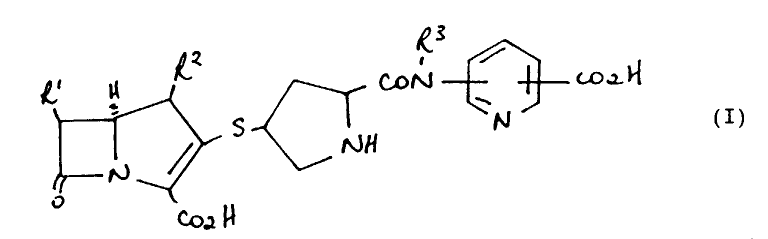 wherein:
R¹ is 1-hydroxyethyl, 1-fluoroethyl or hydroxymethyl;
R² is hydrogen or C₁₋₄alkyl;
R³ is hydrogen or C₁₋₄alkyl;
and the pyridyl group is bonded to the nitrogen of the linking carbamoyl group by a carbon atom, is substituted with the carboxy group on a carbon atom, and is optionally further substituted, on ring carbon atoms, by one or two substituents selected from halo, cyano, C₁₋₄alkyl, nitro, hydroxy, carboxy, C₁₋₄alkoxy, trifluoromethyl, C₁₋₄alkoxycarbonyl, amino, C₁₋₄alkylamino, di-C₁₋₄alkylamino, C₁₋₄alkylS(O)ₙ- (wherein n is 0-2), C₁₋₄alkylamino, C₁₋₄alkanoyl(N-C₁₋₄alkyl)amino, carbamoyl, C₁₋₄alkylcarbamoyl and di-C₁₋₄alkylcarbamoyl:
or a pharmaceutically acceptable salt or in vivo hydrolysable ester thereof.

2. A compound according to claim 1 wherein R¹ is 1-hydroxyethyl and R² is methyl.

3. A compound according to either claim 1 or claim 2 of the formula (IV): 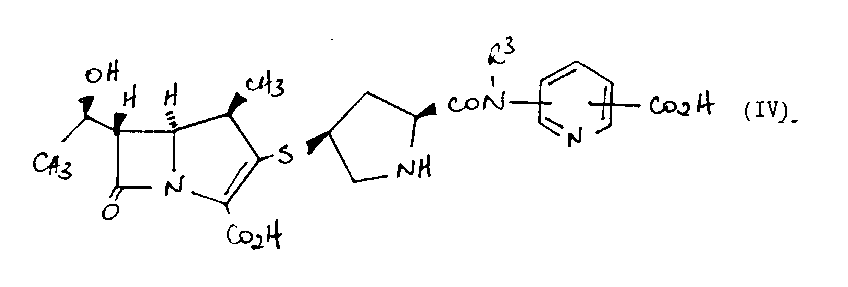 wherein R³ and optional substituents on the pyridyl ring are as defined in claim 1.

4. A compound according to either claim 1 or claim 2 of the formula (IVA): 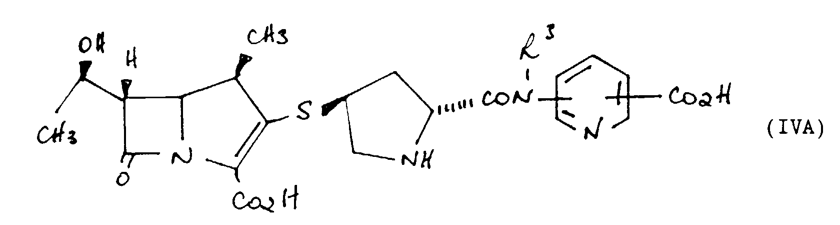 wherein R³ and optional substituents on the pyridyl ring are as defined in claim 1.

5. A compound according to claim 3 wherein optional substituents on pyridyl ring are selected from halo, cyano, C₁₋₄alkyl, nitro, hydroxy, carboxy, C₁₋₄alkoxy, carbamoyl, amino and trifluoromethyl.

6. A compound according to claim 1 which is
(1R,5S,6S,8R,2'S,4'S)-2-(2-(4-carboxy-2-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(3-carboxy-5-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(3-carboxy-2-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(5-carboxy-2-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxy-4-pyridylcarbamoyl)pyrrolidin-4-ylthio)-6-(1-hydroxyethyl)-1-methylcarbapenem-3-carboxylic acid;
(1R,5S,6S,8R,2'S,4'S)-2-(2-(2-carboxy-6-pyridylcarbamoyl)pyrrolidin-4-ylthio)-6-(1-hydroxyethyl)-1-methylcarbapenem-3-carboxylic acid;
(1R,5S,6S,8R,2'R,4'S)-2-(2-(2-carboxy-6-pyridylcarbamoyl)pyrrolidin-4-ylthio)-6-(1-hydroxyethyl)-1-methylcarbapenem-3-carboxylic acid;
and pharmaceutically acceptable salts thereof.

7. A pharmaceutical composition which comprises a compound according to any one of claims 1 to 6 and a pharmaceutically acceptable carrier.

8. A process for preparing a compound according to claim 1 which comprises deprotecting a compound of the formula (V): 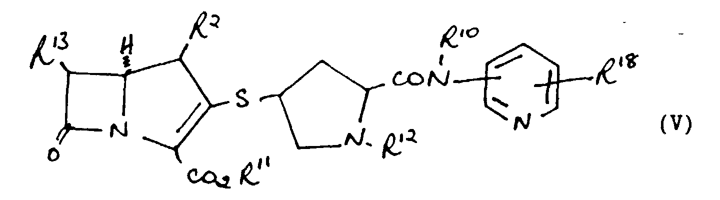 wherein R² is as defined in claim 1; R¹⁰ is a group R³ (as defined in claim 1) or an amino protecting group; R¹³ is a group R¹ (as defined in claim 1), protected hydroxymethyl or 1-(protected hydroxy)ethyl; R¹¹ is hydrogen or a carboxy protecting group; R¹² is hydrogen or an amino protecting group; and
R¹⁸ is carboxy or a protected carboxy group and wherein any optional substituent on the pyridyl ring is as defined in claim 1 and is optionally protected; and wherein at least one protecting group is present; and thereinafter if necessary;
(i) forming a pharmaceutically acceptable salt,
(ii) esterifying to form an in vivo hydrolysable ester.

9. A process for preparing a compound according to claim 1 or a compound of the formula (V) as defined in claim 8 which comprises:
a) reacting compounds of the formulae (VI) and (VII): 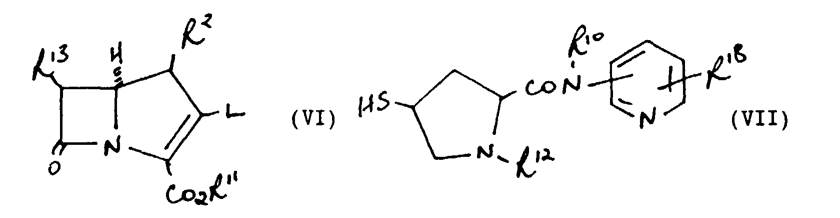 wherein R², R¹⁰, R¹¹ R¹², R¹³ and R¹⁸ are as defined in claim 8, optional substituents on the pyridyl ring are as defined in claim 8 and L is a leaving group, or
b) cyclising a compound of the formula (VIII): 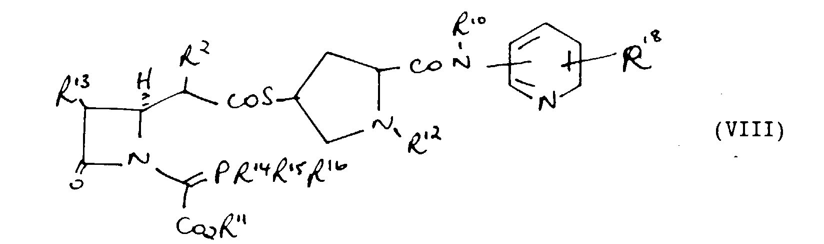 wherein R², R¹⁰, R¹¹, R¹², R¹³ and R¹⁸ are as defined in claim 8, optional substituents on the pyridyl ring are as defined in claim 8 and R¹⁴, R¹⁵ and R¹⁶ are independently selected from C₁₋₆alkoxy, aryloxy, di-C₁₋₆alkylamino and diarylamino or any two of R¹⁴-R¹⁶ represent o-phenylenedioxy or one of R¹⁴-R¹⁶ is C₁₋₄alkyl, allyl, benzyl or phenyl and the other two values are independently selected from C₁₋₄alkyl, trifluoromethyl or phenyl, wherein any phenyl group is optionally substituted with C₁₋₃alkyl or C₁₋₃alkoxy; and wherein any functional group is optionally protected and thereinafter if necessary:
(i) removing any protecting groups;
(ii) forming a pharmaceutically acceptable salt;
(iii) esterifying to form an in vivo hydrolysable ester.

10. A compound of the formula (V) as defined in claim 8, of the formula (VII) or (VIII) as defined in claim 9, or of the formula (IX), (XII) or (XIV): 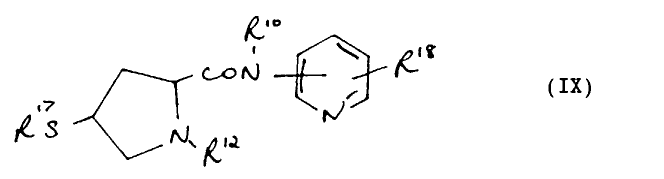 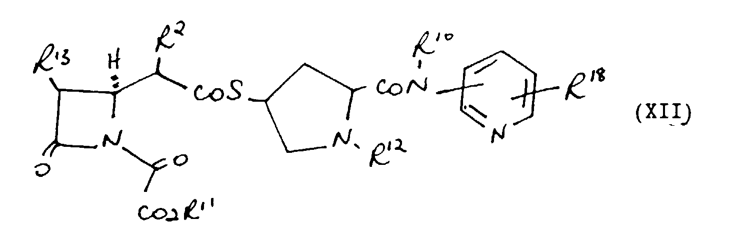 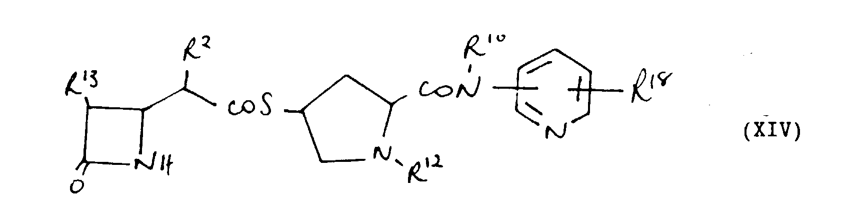 wherein R², R¹⁰-R¹³ and R¹⁸ are as defined in claim 8 and R¹⁷ is a protecting group.

## Patentansprüche

1. Verbindung der Formel (I): 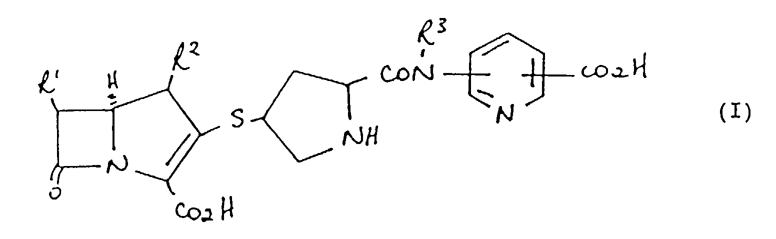 worin:
R¹ 1-Hydroxyethyl, 1-Fluorethyl oder Hydroxymethyl ist;
R² Wasserstoff oder C₁₋₄-Alkyl ist;
R³ Wasserstoff oder C₁₋₄-Alkyl ist;
und die Pyrdyl-Gruppe mit dem Stickstoff der verbindenden Carbamoyl-Gruppe durch ein Kohlenstoffatom verbunden ist, mit der Carboxy-Gruppe an einem Kohlenstoffatom substituiert ist und gegebenenfalls weiter an Ringkohlenstoffatomen durch einen oder zwei Substituenten substituiert ist, welche ausgewählt werden aus Halo, Cyano, C₁₋₄-Alkyl, Nitro, Hydroxy, Carboxy, C₁₋₄-Alkoxy, Trifluormethyl, C₁₋₄-Alkoxycarbonyl, Amino, C₁₋₄-Alkylamino, Di-C₁₋₄-alkylamino, C₁₋₄-Alkyl-S(O)ₙ- (wobei n 0 bis 2 ist), C₁₋₄-Alkylamino, C₁₋₄-Alkanoyl(N-C₁₋₄-alkyl)amino, Carbamoyl, C₁₋₄-Alkylcarbamoyl und Di-C₁₋₄-alkylcarbamoyl;
oder ein pharmazeutisch akzeptables Salz oder ein in vivo hydrolysierbarer Ester davon.

2. Verbindung gemäß Anspruch 1, worin R¹ 1-Hydroxyethyl ist und R² Methyl ist.

3. Verbindung gemäß Anspruch 1 oder Anspruch 2 mit der Formel (IV): 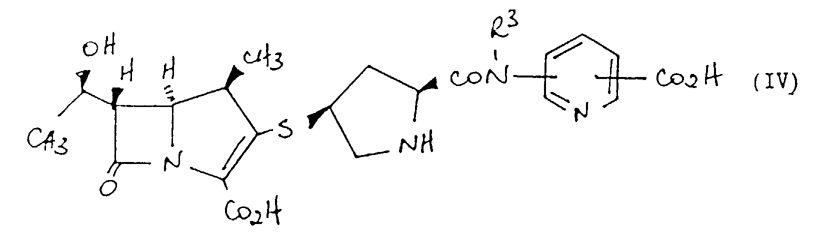 worin R³ und optionale Substituenten am Pyridyl-Ring wie in Anspruch 1 definiert sind.

4. Verbindung gemäß Anspruch 1 oder Anspruch 2 mit der Formel (IVA): 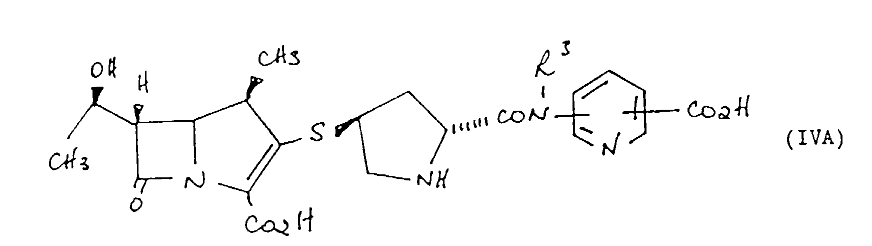 worin R³ und optionale Substituenten am Pyridyl-Ring wie in Anspruch 1 definiert sind.

5. Verbindung gemäß Anspruch 3, worin optionale Substituenten am Pyridyl-Ring ausgewählt werden aus Halo, Cyano, C₁₋₄-Alkyl, Nitro, Hydroxy, Carboxy, C₁₋₄-Alkoxy, Carbamoyl, Amino und Trifluormethyl.

6. Verbindung gemäß Anspruch 1, welche darstellt:
(1R,5S,6S,8R,2'S,4'S)-2-(2-(4-Carboxy-2-pyridylcarbamoyl)pyrrolidin-4-ylthio)-6-(1-hydroxyethyl)-1-methylcarbapenem-3-carbonsäure;
(1R,5S,6S,8R,2'S,4'S)-2-(2-(3-Carboxy-5-pyridylcarbamoyl)pyrrolidin-4-ylthio)-6-(1-hydroxyethyl)-1-methylcarbapenem-3-carbonsäure;
(1R,5S,6S,8R,2'S,4'S)-2-(2-(3-Carboxy-2-pyridylcarbamoyl)pyrrolidin-4-ylthio)-6-(1-hydroxyethyl)-1-methylcarbapenem-3-carbonsäure;
(1R,5S,6S,8R,2'S,4'S)-2-(2-(5-Carboxy-2-pyridylcarbamoyl)pyrrolidin-4-ylthio)-6-(1-hydroxyethyl)-1-methylcarbapenem-3-carbonsäure;
(1R,5S,6S,8R,2'S,4'S)-2-(2-(2-Carboxy-4-pyridylcarbamoyl)pyrrolidin-4-ylthio)-6-(1-hydroxyethyl)-1-methylcarbapenem-3-carbonsäure;
(1R,5S,6S,8R,2'S,4'S)-2-(2-(2-Carboxy-6-pyridylcarbamoyl)pyrrolidin-4-ylthio)-6-(1-hydroxyethyl)-1-methylcarbapenem-3-carbonsäure;
(1R,5S,6S,8R,2'R,4'S)-2-(2-(2-Carboxy-6-pyridylcarbamoyl)pyrrolidin-4-ylthio)-6-(1-hydroxyethyl)-1-methylcarbapenem-3-carbonsäure;
und pharmazeutisch akzeptable Salze davon.

7. Pharmazeutische Zusammensetzung, umfassend eine Verbindung gemäß einem der Ansprüche 1 bis 6 und einen pharmazeutisch akzeptablen Träger.

8. Verfahren zur Herstellung einer Verbindung gemäß Anspruch 1, umfassend die Entschützung einer Verbindung der Formel (V): 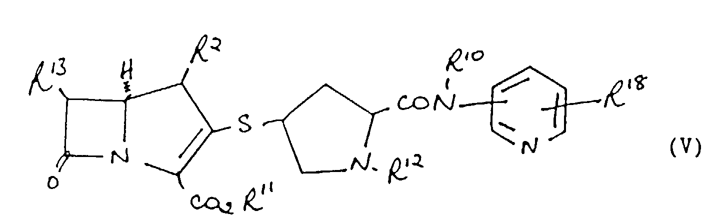 worin
R² wie in Anspruch 1 definiert ist; R¹⁰ eine Gruppe R³ (wie definiert in Anspruch 1) oder eine Amino-Schutzgruppe ist; R¹³ eine Gruppe R¹ (wie definiert in Anspruch 1), geschütztes Hydroxymethyl oder 1-(geschütztes Hydroxy)ethyl ist; R¹¹ Wasserstoff oder eine Carboxy-Schutzgruppe ist; R¹² Wasserstoff oder eine Amino-Schutzgruppe ist; und
R¹⁸ Carboxy oder eine geschützte Carboxy-Gruppe ist, und wobei beliebige optionale Substituenten am Pyridyl-Ring wie in Anspruch 1 definiert sind und gegebenenfalls geschützt sind; und worin wenigstens eine Schutzgruppe vorliegt; und danach, falls erforderlich
(i) Bilden eines pharmazeutisch akzeptablen Salzes,
(ii) Veresterung zur Bildung eines in vivo hydrolysierbaren Esters.

9. Verfahren zur Herstellung einer Verbindung gemäß Anspruch 1 oder einer Verbindung der Formel (V) gemäß Anspruch 8, welches umfaßt:
a) Umsetzung der Verbindungen der Formeln (VI) und (VII): 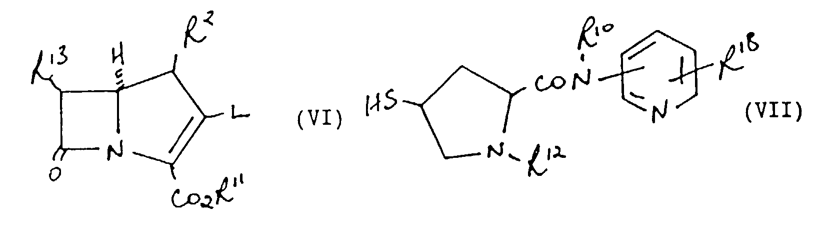 worin R², R¹⁰, R¹¹, R¹², R¹³ und R¹⁸ wie in Anspruch 8 definiert sind, optionale Substituenten am Pyridyl-Ring wie in Anspruch 8 definiert sind, L eine Abgangsgruppe ist, oder
b) Cyclisierung einer Verbindung der Formel (VIII): 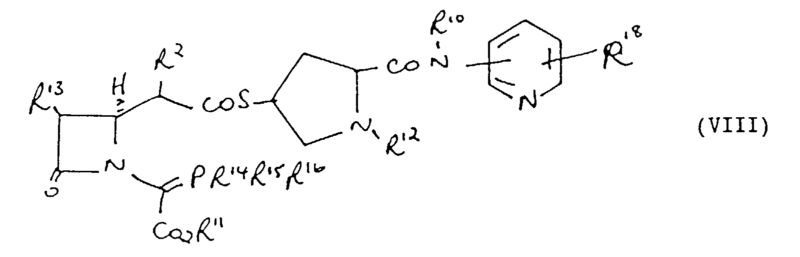 worin R², R¹⁰, R¹¹, R¹², R¹³ und R¹⁸ wie in Anspruch 8 definiert ist, optionale Substituenten am Pyridyl-Ring wie in Anspruch 8 definiert sind und R¹⁴, R¹⁵ und R¹⁶ unabhängig ausgewählt werden aus C₁₋₆-Alkoxy, Aryloxy, Di-C₁₋₄-alkylamino und Diarylamino oder bliebige zwei Gruppen aus R¹⁴-R¹⁶ o-Phenylendioxy darstellen oder eine Gruppe aus R¹⁴-R¹⁶ C₁₋₄-Alkyl,-Allyl, Benzyl oder Phenyl ist und die beiden anderen Werte unabhängig ausgewählt werden aus C₁₋₄-Alkyl, Trifluormethyl oder Phenyl, wobei beliebige Phenyl-Gruppen gegebenenfalls mit C₁₋₃-Alkyl oder C₁₋₃-Alkoxy substituiert sind; und wobei funktionelle Gruppen gegebenenfalls geschützt sind, und danach, falls erforderlich:
(i) Entfernung etwaiger Schutzgruppen;
(ii) Bildung eines pharmazeutisch akzeptablen Salzes;
(iii) Veresterung zur Bildung eines in vivo hydrolysierbaren Esters.

10. Verbindung mit der Formel (V) gemäß Anspruch 8, mit der Formel (VII) oder (VIII) gemäß Anspruch 9, oder mit der Formel (IX), (XII) oder (XIV): 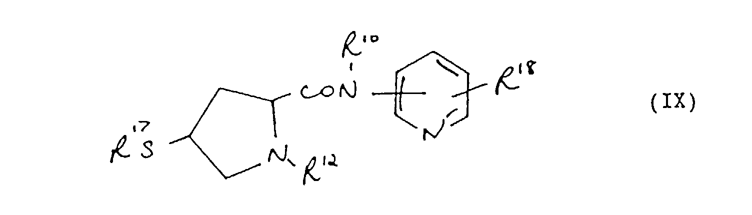 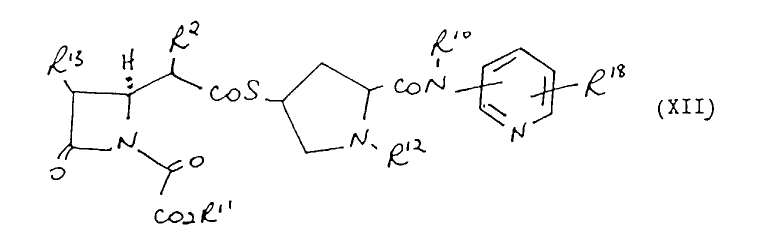 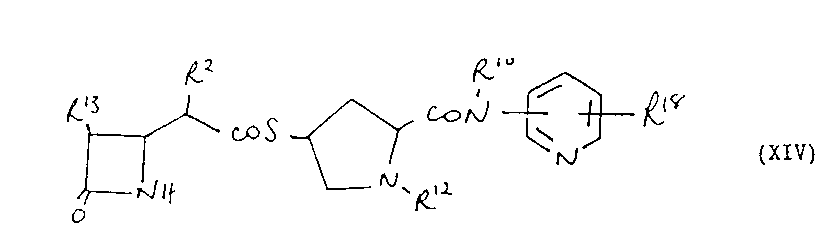 worin R², R¹⁰-R¹³ und R¹⁸ wie in Anspruch 8 definiert sind, und R¹⁷ eine Schutzgruppe ist.

## Revendications

1. Composé de formule (I) : 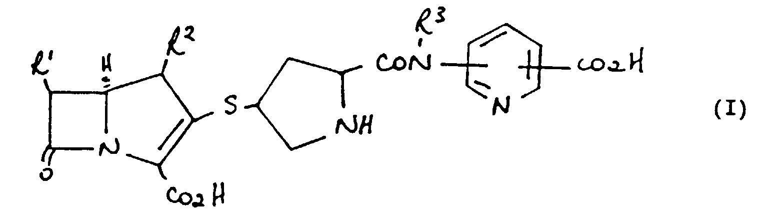 dans laquelle :
- R¹ est un groupe 1-hydroxyéthyle, 1-fluoroéthyle ou hydroxyméthyle ;
- R² est un atome d'hydrogène ou un groupe alkyle C₁₋₄ ;
- R³ est un atome d'hydrogène ou un groupe alkyle C₁₋₄ ; et
le groupe pyridyle est lié à l'azote du groupe de liaison carbamoyle par un atome de carbone, est substitué par le groupe carboxy sur un atome de carbone et est éventuellement substitué aussi sur les atomes de carbone du cycle par des groupes halo, cyano, alkyle C_{1-4,} nitro, hydroxy, carboxy, alcoxy C₁₋₄, trifluorométhyle, alcoxy(C₁₋₄)carbonyle, amino, alkyl(C₁₋₄)amino, dialkyl(C₁₋₄)amino, alkyl(C₁₋₄)-S(O)ₙ (dans lequel n varie de 0 à 2), alkyl(C₁₋₄)amino, alcanoyl(C₁₋₄)-[N-alkyl(C₁₋₄)]amino, carbamoyle, alkyl(C₁₋₄)carbamoyle et dialkyl(C₁₋₄)carbamoyle ;
ou sel pharmaceutiquement acceptable de celui-ci ou ester pharmaceutiquement acceptable de celui-ci, hydrolysable in vivo.

2. Composé selon la revendication 1, dans lequel R¹ est un groupe 1-hydroxyéthyle et R² est un groupe méthyle.

3. Composé selon la revendication 1 ou la revendication 2, de formule (IV) : 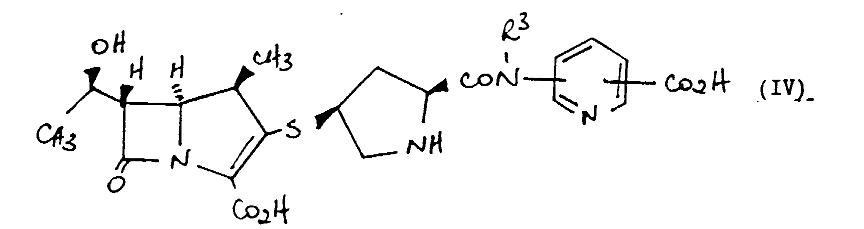 dans laquelle R³ et les substituants éventuels sur le cycle pyridyle sont tels que définis dans la revendication 1.

4. Composé selon la revendication 1 ou la revendication 2, de formule (IVA): 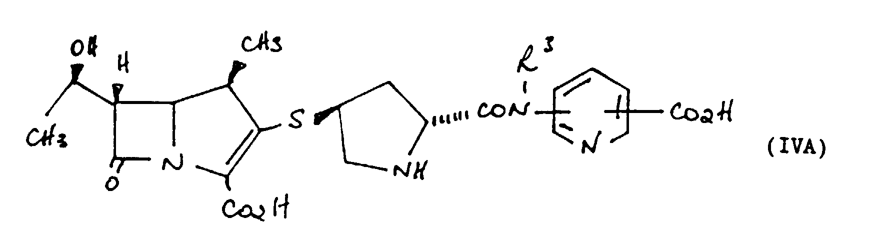 dans laquelle R³ et les substituants éventuels sur le cycle pyridyle sont tels que définis dans la revendication 1.

5. Composé selon la revendication 3 dans lequel les substituants éventuels sur le cycle pyridyle sont choisis parmi les groupes halo, cyano, alkyl C₁₋₄, nitro, hydroxy, carboxy, alcoxy C_{1-4,} carbamoyle, amino et trifluorométhyle.

6. Composé selon la revendication 1, qui est :
- l'acide (1R, 5S, 6S, 8R, 2'S, 4'S)-2-[2-(4-carboxy-2-pyridylcarbamoyl)-pyrrolidin-4-ylthio]-6-(1-hydroxyéthyl)-1-méthylcarbapénème-3-carboxylique ;
- l'acide (1R, 5S, 6S, 8R, 2'S, 4'S)-2-[2-(3-carboxy-5-pyridylcarbamoyl)-pyrrolidin-4-ylthio]-6-(1-hydroxyèthyl)-1-méthylcarbapénème-3-carboxylique ;
- l'acide (1R, 5S, 6S, 8R, 2'S, 4'S)-2-[2-(3-carboxy-2-pyridylcarbamoyl)-pyrrolidin-4-ylthio]-6-(1-hydroxyéthyl)-1-méthylcarbapénème-3-carboxylique ;
- l'acide (1R, 5S, 6S, 8R, 2'S, 4'S)-2-[2-(5-carboxy-2-pyridylcarbamoyl)-pyrrolidin-4-ylthio]-6-(1-hydroxyéthyl)-1-méthylcarbapénème-3-carboxylique ;
- l'acide (1R, 5S, 6S, 8R, 2'S, 4'S)-2-[2-(2-carboxy-4-pyridylcarbamoyl)-pyrrolidin-4-ylthio]-6-(1-hydroxyéthyl)-1-méthylcarbapénème-3-carboxylique ;
- l'acide (1R, 5S, 6S, 8R, 2'S, 4'S)-2-[2-(2-carboxy-6-pyridylcarbamoyl)-pyrrolidin-4-ylthio]-6-(1-hydroxyéthyl)-1-méthylcarbapénéme-3-carboxylique ;
- l'acide (1R, 5S, 6S, 8R, 2'R, 4'S)-2-[2-(2-carboxy-6-pyridylcarbamoyl)-pyrrolidin-4-ylthio]-6-(1-hydroxyéthyl)-1-méthylcarbapénème-3-carboxylique ;
et les sels pharmaceutiquement acceptables de ceux-ci.

7. Composition pharmaceutique qui comprend un composé selon l'une quelconque des revendications 1 à 6 et un véhicule pharmaceutiquement acceptable.

8. Procédé de préparation d'un composé selon la revendication 1, qui comprend la déprotection d'un composé de formule (V): 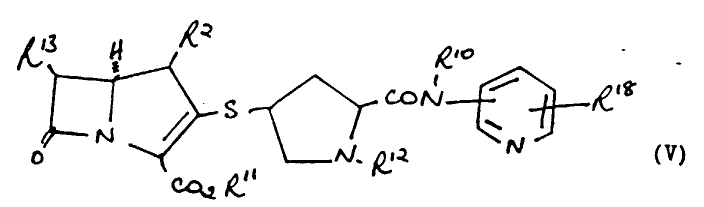 dans laquelle R² est tel que défini dans la revendication 1 ; R¹⁰ est un groupe R³ (tel que défini dans la revendication 1) ou un groupe protecteur de groupe amino ; R¹³ est un groupe R¹ (tel que défini dans la revendication 1), un groupe hydroxyméthyle protégé ou un groupe 1-(hydroxy protégé)éthyle ; R¹ est un atome d'hydrogène ou un groupe protecteur de groupe carboxy ; R¹² est un atome d'hydrogène ou un groupe protecteur de groupe amino ; et R¹⁸ est un groupe carboxy ou carboxy protégé,
et dans laquelle au moins un groupe protecteur est présent ; et ensuite si nécessaire ;
(i) la formation d'un sel pharmaceutiquement acceptable,
(ii) l'estérification pour former un ester hydrolysable in vivo.

9. Procédé de préparation d'un composé selon la revendication 1, ou composé de formule (V) tel que défini dans la revendication 8, qui comprend :
(a) la réaction de composés de formules (VI) et (VII): 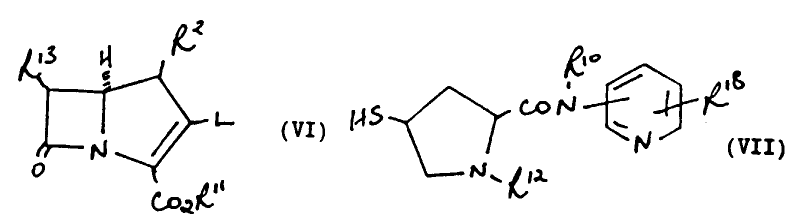 dans lesquelles R², R¹⁰, R¹¹, R¹², R¹³ et R¹⁸ sont tels que définis dans la revendication 8, les substituants éventuels sur le cycle pyridyle sont tels que définis dans la revendication 8 et L est un groupe partant, ou
(b) la cyclisation d'un composé de formule (VIII) : 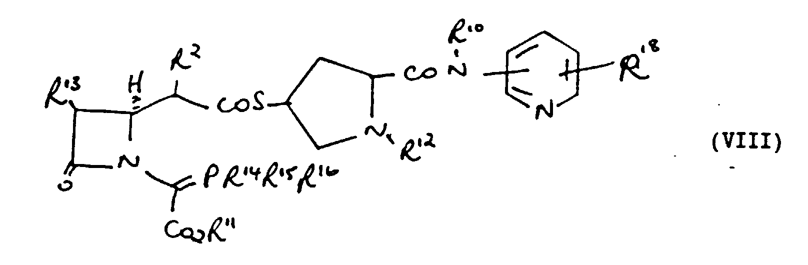 dans laquelle R², R¹⁰, R¹¹, R¹², R¹³ et R¹⁸ sont tels que définis dans la revendication 8, les substituants éventuels sur le cycle pyridyle sont tels que définis dans la revendication 8 et R¹⁴, R¹⁵ et R¹⁶ sont choisis indépendamment parmi les groupes alcoxy C₁₋₆, aryloxy, dialkyl(C₁₋₆)-amino et diarylamino ou bien deux quelconques de R¹⁴-R¹⁶ représentent un groupe o-phénylènedioxy ou l'un de R¹⁴-R¹⁶ est un groupe alkyl C₁₋₄, allyle, benzyle ou phényle et les deux autres sont choisis indépendamment parmi les groupes alkyle C_{1-4,} trifluorométhyle ou phényle, tout groupe phényle étant éventuellement substitué par des groupes alkyle C₁₋₃ ou alcoxy C₁₋₃; et dans laquelle tout groupe fonctionnel est éventuellement protégé, et ensuite si nécessaire :
(i) l'élimination de tout groupe protecteur ;
(ii) la formation d'un sel pharmaceutiquement acceptable,
(iii) l'estérification pour former un ester hydrolysable in vivo.

10. Composé de formule (V) tel que défini dans la revendication 8, de formule (VII) ou (VII) tel que défini dans la revendication 9 ou de formule (IX), (XII) ou (XIV) : 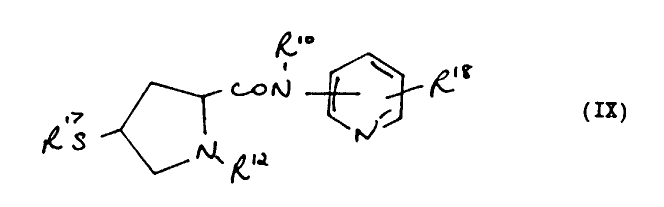 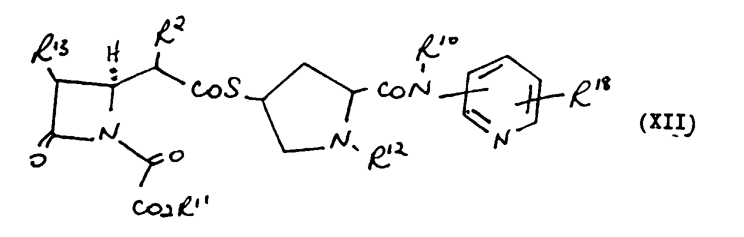 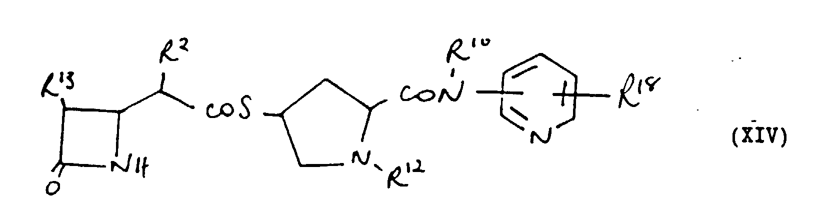 dans lesquelles R², R¹⁰-R¹³ et R¹⁸ sont tels que définis dans la revendication 8 et R¹⁷est un groupe protecteur.
